# EUROPEAN PATENT APPLICATION

(11) **EP 3 900 727 A1**
(43) Date of publication of application: **27.10.2021**
(21) Application number: 19901170.1
(22) Date of filing: 15.11.2019
(51) Int. Cl.: A61K 31/519, A61P 1/00

(54) **IMMUNITY MECHANISM AND THERAPEUTIC DRUG FOR GASTROINTESTINAL DISEASES**

(30) Priority: 21.12.2018 CN 201811573243; 17.10.2019 CN 201910988111
(71) Applicant: Guangzhou Women and Children's Medical Center, Guangzhou, Guangdong 510623 (CN)
(72) Inventor: ZHANG, Yuxia, Guangzhou, Guangdong 510623 (CN); YANG, Min, Guangzhou, Guangdong 510623 (CN); HUANG, Bing, Guangzhou, Guangdong 510623 (CN); CHEN, Zhanghua, Guangzhou, Guangdong 510623 (CN); ZHANG, Li, Guangzhou, Guangdong 510623 (CN); BAI, Fan, Guangzhou, Guangdong 510623 (CN); GONG, Sitang, Guangzhou, Guangdong 510623 (CN)
(74) Representative: karo IP
(86) International application number: PCT/CN2019/118875
(87) International publication number: WO 2020/125293

(57) **Abstract**

The present invention relates to gastrointestinal diseases, such as non-infectious gastrointestinal diseases, including the immune mechanism of non-infectious gastrointestinal diseases in children, and the use of phosphodiesterase inhibitors and/or antiplatelet drugs in the treatment of gastrointestinal diseases such as non-infectious gastrointestinal diseases.

## Description

### Technical field

The present invention relates to gastrointestinal diseases, such as infectious or non-infectious gastrointestinal disease, especially non-infectious gastrointestinal disease, including the immune mechanism of children's non-infectious gastrointestinal disease. The present invention also relates to the new use of PDE and/or antiplatelet drugs for treating gastrointestinal diseases, such as non-infectious gastrointestinal diseases.

### Background technique

Gastrointestinal diseases, such as non-infectious gastrointestinal diseases, is a general term for a variety of inflammatory diseases of the digestive tract. According to different classification angles, including, for example, inflammatory bowel diseases (such as inflammatory bowel disease, Sometimes referred to as IBD)), allergic gastrointestinal diseases (such as food allergies), and functional gastrointestinal diseases, etc., can affect people of all ages. It is also especially common in infants and young children, and is a major medical problem that affects the healthy growth of children. The clinical manifestations are repeated abdominal pain, abdominal distension, hematemesis, blood in the stool, diarrhea, constipation, loss of appetite, malnutrition, and various systemic complications, which can lead to death in severe cases. The pathogenesis of gastrointestinal diseases, such as non-infectious gastrointestinal diseases, is still unclear. This is reflected in the fact that the naming of disease subtypes is mainly based on the affected site, clinical manifestations, predisposing factors or the number of a certain cell type rather than the pathogenesis To proceed. With the development of society and economy, gastrointestinal diseases, especially inflammatory bowel disease, include, for example, undifferentiated colitis (undifferentiated colitis), Crohn's disease (hereinafter sometimes referred to as CD) and ulcerative colitis (The incidence of Ulcerative colitis (hereinafter sometimes referred to as UC) is increasing year by year worldwide, and it is also a common clinical disease in China. At present, the treatment of gastrointestinal diseases is only symptomatic treatment based on the possible causes. The methods include allergen avoidance, enteral nutrition, application of glucocorticoids, immunosuppressive agents and biological agents to inhibit inflammation. Long-term use of these drugs may cause side effects that affect organ function, increase the risk of infection, and may induce the risk of malignant tumors such as lymphoma. Especially for children, these risks have a significant impact on children in the growth and development period.

Among them, in terms of allergic gastrointestinal diseases, the incidence of food allergies has increased year by year in the past 20 years, because the immune response mechanism is not fully understood, and due to the lack of reliable diagnostic methods, the resulting gastrointestinal diseases such as Food protein-induced enterocolitis syndrome, food protein-induced enteropathy, eosinophilic esophagitis, eosinophilic gastroenteritis and many other diseases are often delayed or misdiagnosed and mistreated. The management of such children depends on individualized allergen avoidance, prevention or avoidance of acute and chronic symptoms caused by allergic foods, and an adequate and balanced diet. However, the dietary avoidance management of such children is very difficult. Too broad or too strict may lead to repeated acute allergic reactions or continuous gastrointestinal inflammation that directly affect the digestion and absorption of nutrients.

In addition, in terms of functional gastrointestinal diseases, in the past 10 years, children's functional gastrointestinal diseases caused by abnormal brain-gut interaction have attracted more and more attention. The occurrence of symptoms is related to gastrointestinal motility disorders and high sensitivity of internal organs. Sex, mucosal and immune dysfunction, imbalance of intestinal flora, and abnormal processing of the central nervous system are related to factors. Due to the lack of understanding of the etiology and pathogenesis, the treatment of children with functional gastrointestinal diseases lacks specific treatment methods.

Gastrointestinal diseases such as non-infectious gastrointestinal diseases, which lead to acute attacks and persistent chronic subclinical inflammatory reactions or repeated attacks of the disease, seriously affect the health and growth of the majority of patients, especially children, to the family and society It also brings a huge economic burden. Limited to the source of tissues and the complexity of clinical basic research, the pathogenesis of gastrointestinal diseases is mostly limited to the differences in the overall transcriptome level of biopsy tissues between patients and control groups. Research on the function of specific cells or genes in diseases is mostly limited to experimental animal models. Therefore, the systematic study of gastrointestinal diseases such as non-infectious gastrointestinal diseases, the mucosal immune microenvironment including immune cells and non-immune cells, has great clinical and basic research significance

### Summary of the invention

If the differences in the gastrointestinal immune microenvironment between normal and gastrointestinal diseases such as non-infectious gastrointestinal diseases can be explained at the cellular and molecular levels, it will greatly improve the clinical diagnosis and treatment status of such diseases, and reduce family and social Medical burden, and promote the harmonious development of society.

In order to solve the above-mentioned problems, the present inventors conducted in-depth research on the immune mechanism of gastrointestinal diseases such as non-infectious gastrointestinal diseases.

The present invention selects representative examples of gastrointestinal diseases, and analyzes children and healthy controls suffering from inflammatory bowel diseases, including undifferentiated colitis, Crohn's disease and ulcerative colitis at the single-cell level. The transcriptional profile characteristics of colonic immune and non-immune cells in child subjects proved that CD8 and γδ T cells expressing CD39 (encoded by ENTPD1) are used in the Intraepithelial T cell (Intraepithelial T cell) of patients with inflammatory bowel disease. IET) expression is reduced, which leads to platelet aggregation and serotonin (sometimes referred to as 5-Hydroxytrptamine or 5-HT hereinafter) in the colonic mucosa. Specifically, the present invention uses single-cell RNA sequencing, immunophenotyping analysis, animal experiments and clinical trials on the intestinal mucosa of patients (such as patients with undifferentiated colitis, Crohn's disease and ulcerative colitis) and healthy control subjects. The experiment explained the common pathogenesis of inflammatory bowel disease. The above-mentioned various analyses performed by the present invention show that the cyclic AMP response (cAMP response, cyclic adenosine monophosphate response) and/or the cyclic GMP response (cGMP respopnse, cyclic guanosine monophosphate response) pathway regulates the expression of CD39, and that in patients with inflammatory bowel disease There are pathogenesis caused by defective cAMP and/or cGMP response pathways. Compared with control subjects, in patients with inflammatory bowel disease, macrophages expressing PDE4B and TNFα are infiltrated; the abundance of CD39-expressing intraepithelial T cells (hereinafter sometimes referred to as CD39+IET cells) is reduced, and generally There is platelet aggregation and serotonin release in the colonic mucosa. It can be seen that in patients with inflammatory bowel disease (such as colitis, UC and CD), there are high-level inflammatory macrophage infiltration, CD39+IET deficiency and platelet aggregation defects, and defective cAMP and/or cGMP responses The way is that the common mechanism works.

As mentioned above, the system of the present invention explains the pathogenesis of gastrointestinal diseases and provides new ideas for the treatment of gastrointestinal diseases. As a treatment targeting this pathogenesis, the present invention can improve the immune mechanism by improving the defective cAMP and/or cGMP response pathway, and/or target platelet aggregation for treatment, including administering to patients Drugs or other treatments that can increase cAMP levels and/or cGMP levels, for example, phosphodiesterase (phosphodiesterase, sometimes referred to as PDE) inhibitors that target the cAMP/cGMP pathway, and Adenocine cyclase (Adenocine cyclase) , Hereinafter sometimes referred to as AC) activator, guanylate cyclase (hereinafter sometimes referred to as GC) activator, etc. Specifically, for example, the present inventors found that phosphodiesterase inhibitors, such as Dipyridamole, can inhibit the infiltration of macrophages and reduce their TNF-α expression levels, and can promote CD39 in IET Expression can reduce the aggregation of platelets in the colonic mucosa, and improve the clinical symptoms by restoring the immune homeostasis of patients with inflammatory bowel disease. This provides a new type of treatment for patients suffering from gastrointestinal diseases, such as inflammatory bowel disease, especially colitis, UC and CD.

On the other hand, the present invention also provides ideas for treating gastrointestinal diseases by selecting any one or a combination of the following methods: increasing CD39+IET cells, and/or inhibiting platelet aggregation, and/or inhibiting the release of serotonin , And/or inhibit PDE4B, and/or inhibit TNF-α, and/or inhibit the infiltration of macrophages.

For example, the present inventors discovered that there is colonic mucosal platelet aggregation in patients with gastrointestinal diseases. The present invention can treat gastrointestinal diseases by inhibiting platelet aggregation. As a platelet-targeted treatment, it includes administering drugs or other treatments that can inhibit platelet aggregation to the patient. By administering such drugs, drugs known to be used for antiplatelet, such as dipyridamole, can treat gastrointestinal diseases in patients. Furthermore, the present invention also discovered that methylprednisolone, a clinically used hormone drug for the treatment of gastrointestinal diseases, also has the effect of inhibiting platelet aggregation. This also further supports the explanation of the present invention that anti-platelet aggregation drugs can be used to treat gastrointestinal diseases.

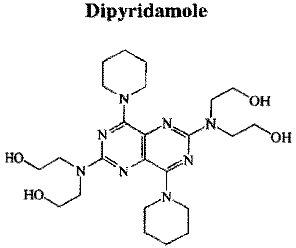

The present invention greatly promotes the precise classification of gastrointestinal diseases such as non-infectious gastrointestinal diseases by explaining the composition and functional state of the local immune microenvironment, thereby improving the level of clinical personalized diagnosis and treatment.

The present invention discovers the pathogenesis of gastrointestinal diseases such as colitis and IBD (including UC and CD, etc.), provides new treatment methods for children, provides ideas for the research and development direction and application of new drugs, and provides some products that have been on the market. Drugs (such as dipyridamole, etc.) provide new therapeutic uses.

As mentioned above, the present invention relates to the following aspects.
1. Use of cAMP and/or cGMP promoters in the preparation of medicines for the treatment and prevention of gastrointestinal diseases.
2. The use according to item 1, wherein the cAMP and/or cGMP promoter is selected from phosphodiesterase inhibitors and adenylate cyclase/guanylate cyclase activators.
3. The use according to item 2, wherein the phosphodiesterase inhibitor is a PDE3, and/or PDE4, and/or PDE5 inhibitor.
4. The use according to item 3, wherein the phosphodiesterase inhibitor is dipyridamole.
5. Use of antiplatelet drugs in the preparation of drugs for the treatment and prevention of gastrointestinal diseases.
6. The use according to item 5, wherein the antiplatelet agent is selected from the group consisting of thromboxane A2 (TXA2) inhibitors, adenosine diphosphate (ADP) P2Y12 receptor antagonists, thrombin receptor antagonists, 5 -Serotonin (5-HT) receptor antagonist, platelet glycoprotein (GP) II b/IIIa receptor inhibitor, and phosphodiesterase inhibitor.
7. The use according to item 6, wherein the antiplatelet drug is a phosphodiesterase inhibitor.
8. The use according to item 7, wherein the antiplatelet drug is dipyridamole.
9. The use according to any one of the foregoing 1-8, wherein the gastrointestinal disease is selected from the group consisting of inflammatory gastrointestinal disease, allergic gastrointestinal disease, and functional gastrointestinal disease.
10. The use according to any one of the foregoing 1-9, wherein the gastrointestinal disease is selected from the group consisting of inflammatory bowel disease, gastrointestinal disease caused by food allergy, and functional gastrointestinal disease.
11. The use according to any one of the foregoing 1-9, wherein the gastrointestinal disease is selected from the group consisting of colitis, ulcerative colitis, Crohn's disease, eosinophilic colitis, non-eosinophilic colitis Granulocyte colitis.
11. The use according to any one of 1-10, wherein the gastrointestinal disease is a childhood gastrointestinal disease.
12. Use of antiplatelet drugs or cAMP and/or cGMP promoters in combination with other drugs in the preparation of drugs for the treatment of gastrointestinal diseases.
13. The use according to 12, wherein the antiplatelet agent is selected from the group consisting of thromboxane A2 (TXA2) inhibitors, adenosine diphosphate (ADP) P2Y12 receptor antagonists, thrombin receptor antagonists, 5- Serotonin (5-HT) receptor antagonist, platelet glycoprotein (GP) II b/IIIa receptor inhibitor, and phosphodiesterase inhibitor.
14. The use according to 12, wherein the cAMP and/or cGMP promoter is selected from phosphodiesterase inhibitors and adenylate cyclase/guanylate cyclase activators.
15. The use of 13 above, wherein the antiplatelet drug is dipyridamole.
16. The use of 14 above, wherein the cAMP and/or cGMP promoter is a PDE3, and/or PDE4, and/or PDE5 inhibitor.
17. The use according to any one of the foregoing 12-16, wherein the gastrointestinal disease is selected from the group consisting of inflammatory gastrointestinal disease, allergic gastrointestinal disease, and functional gastrointestinal disease.
18. The use of any one of the foregoing 12-17, wherein the gastrointestinal disease is selected from ulcerative colitis, Crohn's disease, eosinophilic colitis, non-eosinophilic colitis ,colitis.
19. The use of any one of the foregoing 12-18, wherein the gastrointestinal disease is childhood colitis.
20. A method for diagnosing gastrointestinal diseases, the method comprising: detecting the expression of T cell CD39 and/or the number of platelets, and if the expression of CD39 decreases and/or the number of platelets increases, it is judged as positive
21. A method for diagnosing gastrointestinal diseases, the method comprising: detecting CD39 expression of T cells in the intestinal mucosa of a patient or subject, and/or aggregation of platelets, and/or giants expressing TNF-α and/or PDE4B Infiltration of phages, if the expression of CD39 decreases, and/or the number of platelets increases, and/or the expression of TNF-α increases, it is judged as positive.
22. A kit for diagnosing gastrointestinal diseases, which includes a reagent for detecting whether T cell CD39 expression is decreased, and/or a reagent for detecting whether the number of platelets is decreased.
23. A kit for diagnosing gastrointestinal diseases, comprising: a reagent for detecting CD39 expression of T cells, and/or a reagent for detecting platelet aggregation, and/or a reagent for detecting macrophages expressing TNF-α and/or PDE4B Infiltration reagent.
24. A method for preventing or treating gastrointestinal diseases, the method comprising administering to the patient an effective amount of cAMP and/or cGMP promoter, and/or antiplatelet drug.
25. The method or kit of any one of 20-24, wherein the gastrointestinal disease is selected from the group consisting of inflammatory gastrointestinal disease, allergic gastrointestinal disease, and functional gastrointestinal disease.
26. The method or kit of any one of the aforementioned 20-24, wherein the gastrointestinal disease is selected from the group consisting of colitis, inflammatory bowel disease, ulcerative colitis, Crohn's disease, eosinophilic colitis, non- Eosinophilic colitis.
27. The method or kit of any one of 20-26, wherein the gastrointestinal disease is a non-infectious gastrointestinal disease.
28. The method or kit of any one of 20-27, wherein the patient with gastrointestinal disease is a child.

### Description of the drawings

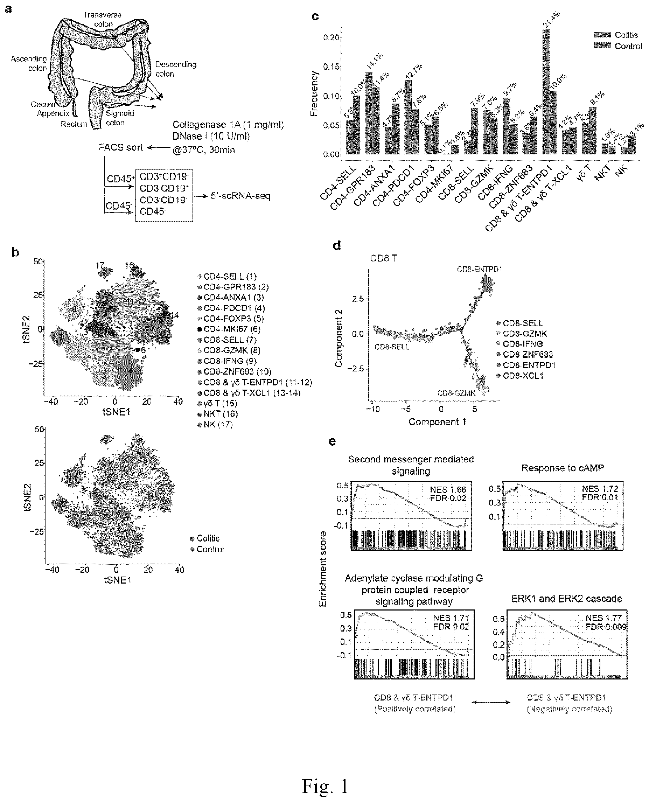

Figure 1: Single cell sequencing and bioinformatics analysis. Among them, Figure (a) shows the colon mucosa of children with colitis (including IBD (including undifferentiated colitis, UC, CD), eosinophilic colitis, and non-eosinophilic colitis patients) and control children's colon mucosa. Figure (b) shows that a total of 17 T and NK subgroups were found by single cell sequencing and bioinformatics analysis. Figure (c) shows the comparison of intraepithelial T cells (IET) in the control group and colitis patients. In each group of histograms, the left side represents the control and the right side represents the colitis group. Figure (d) shows that ENTPD1-CD8 T cells are immunoprotective cell subtypes with specific differentiation characteristics. Figure (e) shows that the differentiation of ENTPD1-CD8 T cells is regulated by the second messenger of cAMP/cGMP. ENTPD1 is the gene encoding CD39.

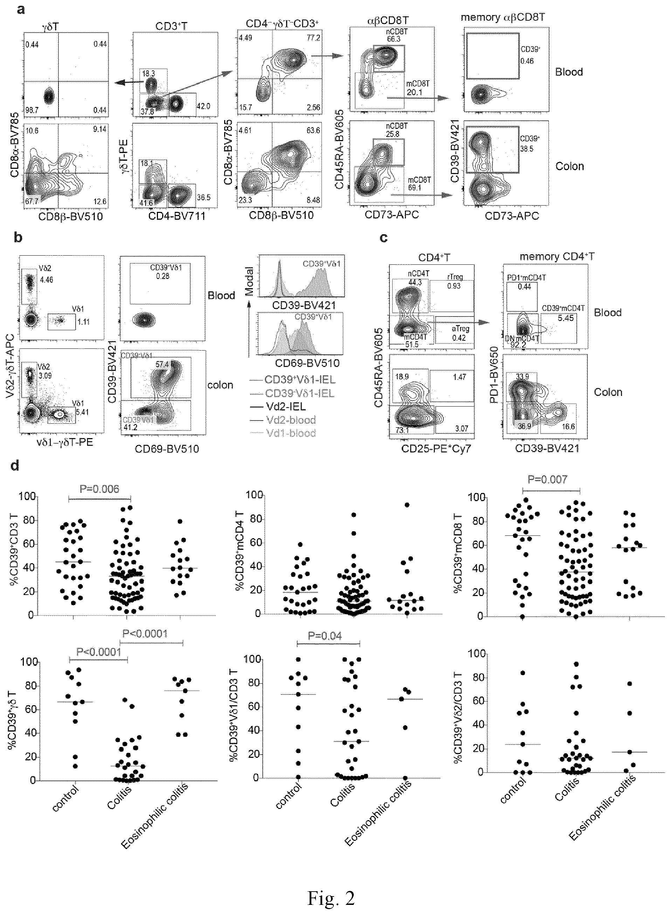

Figure 2: Decreased ratio of CD8 and γδT-ENTPD1 cells in children with colitis. Among them, Figures (a) ~ (c) show the gating strategy and surface marker protein expression in blood and colon intraepithelial T cells (IETs). Panel (d) shows the ratio of designated IETs for control subjects and children with colitis. In children with colitis, CD39+CD3+T cells, especially CD39+memory CD8T cells (CD39+mCD8T) and CD39+γδT cells (Vδ1+) are significantly reduced in proportion. The horizontal line depicts the median. The P value was determined by Kruskal-Wallis test and adjusted for multiple comparisons using Prism 7's Benjamini and Hochberg's original FDR method.

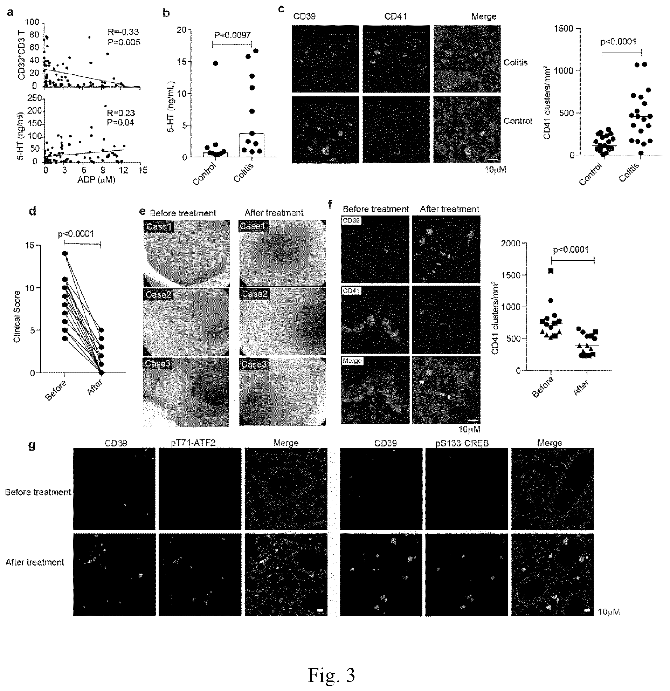

Figure 3: Dimidamol restores the expression of CD39 on T cells and improves the symptoms of colitis in children. Among them, Figure (a) shows the correlation between CD39+CD3+ T cells and ADP and 5-HT released from colon biopsy tissues stored overnight. Prism 7 was used to determine the P value by linear regression. Figure (b) shows that cultured platelets from children with colitis secrete significantly higher amounts of 5-HT than control subjects. The P value was calculated by Mann-Whitney U test (two-sided). In Figure (c), the left panel shows that immunofluorescence staining shows that there is a negative correlation (400x) between CD39 expression and platelet aggregation (CD41 cluster) in the control group and colitis subjects. CD39 (green), CD41 (red), and nuclear staining DAPI (blue). The figure on the right shows that in the control group (n = 3) and colitis subjects (n = 4), the CD41 clusters/mm2 counts as 4-5 areas/colon biopsy (only count the diameter in the image> 7µm) Of those aggregated platelets). The horizontal line depicts the median. The P value was calculated by Mann-Whitney U test (two-sided). Figure (d) shows the clinical scores before and after dipyridamole treatment. P value is calculated by Wilcoxon matching pair signed rank test (two-sided). Figure (e) shows the colonoscopy appearance and clinical score before and after dipyridamole treatment. Figure (f) shows the immunofluorescence results of CD39 and CD41 clusters before and after dipyridamole treatment. The figure on the right shows the number of CD41 clusters in 3 children with colitis before and after dipyridamole treatment. The P value was calculated by Mann-Whitney U test (two-sided). Figure (g) shows a representative immunofluorescence staining (200x) showing the co-expression of activated ATF2 and CREB and CD39 in the colonic mucosa after dipyridamole treatment.

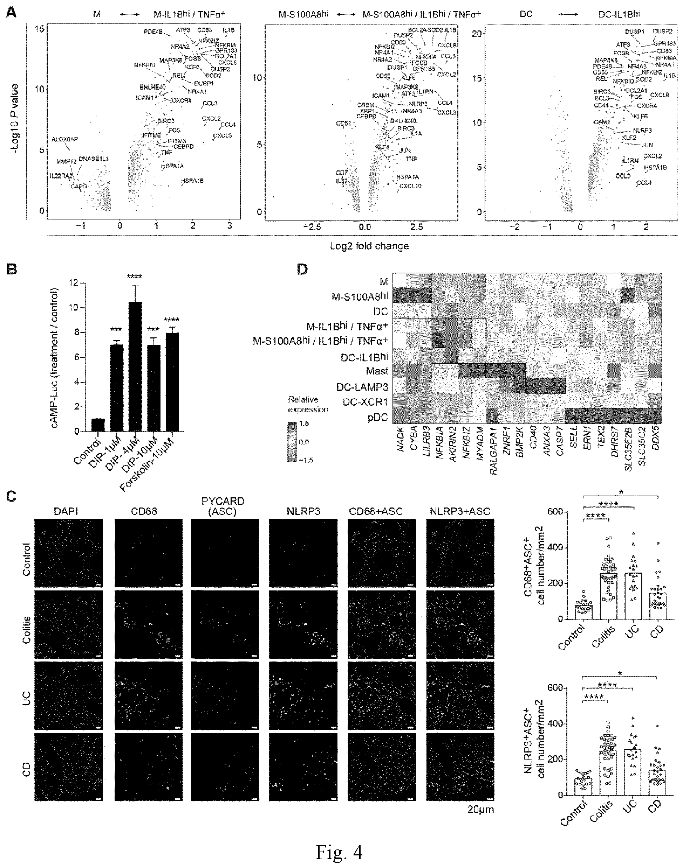

Figure 4: Highly inflammatory macrophage and dendritic cell (DC) infiltration in colitis and IBD patients compared to control subjects. (A) shows the differentially expressed genes (DEG) of highly inflammatory macrophages and DC fractions compared to their control counterparts. The red and navy blue dots represent individual genes that pass the P value (≤0.05) and log2 (Fold Change) (red, ≥ 1; navy blue, ≤-1) threshold. (B) When dipyridamole is applied to HEK 293T cells stably transfected with the cAMP reporter gene GloSensor-22F cAMP plasmid, it can significantly increase the cytoplasmic cAMP concentration. Forskolin, which increases the concentration of cAMP by activating adenylate cyclase, was used as a positive control. The data represents 3 independent experiments, all of which showed similar results. (C) Immunofluorescence staining showed that the NLRP3 inflammasome of the patient and child was activated. Colonic mucosa sections were stained with anti-CD68 to mark macrophages, and stained with NLRP3 and ASC for inflammasome. The right panel shows the quantitative analysis of CD68 + ASC + and NLRP3 + ASC + cells, with eosinophilic colitis shown by blue dots. The P value was calculated using the Mann-Whitney U test, *, P <0.05 ****, P <0.0001. (D) Heat map showing the relative expression of colitis/IBD risk genes in myeloid cells.

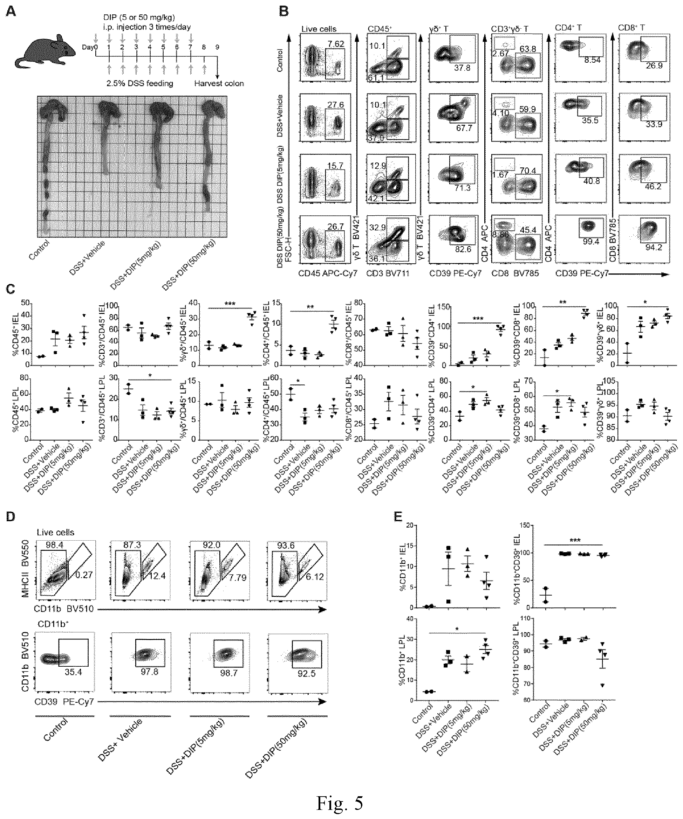

Figure 5: Dipyridamole treatment increases the expression of CD39 in intraepithelial T lymphocytes in a DSS-induced acute colitis model. (A) The figure above shows the treatment plan for the DSS-induced acute colitis model. The figure below represents the colon length of mice in the control group, DSS colitis group, or DSS + dipyridamole group. (B) Intraepithelial T cell subtypes of colitis and the gating strategy of CD39 expression in mice in the control group, DSS colitis group, and DSS + dipyridamole group. (C) The summary data shows that the intraperitoneal injection of dipyridamole at a dose of 50 mg/kg significantly increased the expression of CD39 in CD4, CD8 and γδ T cells. Dipyridamole at 50 mg/kg can increase the relative abundance of CD4 and γδ T cells. Use Mann-Whitney U test to calculate P value, *, P <0.05. **, P <0.01.***, P <0.001. (D) Control group, DSS colitis group, or DSS + dipyridamole group The gating strategy of myeloid cells and the expression of CD39 in mice. (E) The summary data shows the expression of CD39 in myeloid cells of mice in the control group, DSS colitis group, or DSS + dipyridamole group. Use Mann-Whitney U test to calculate P value, *, P <0.05. **, P <0.01.***, P <0.001.

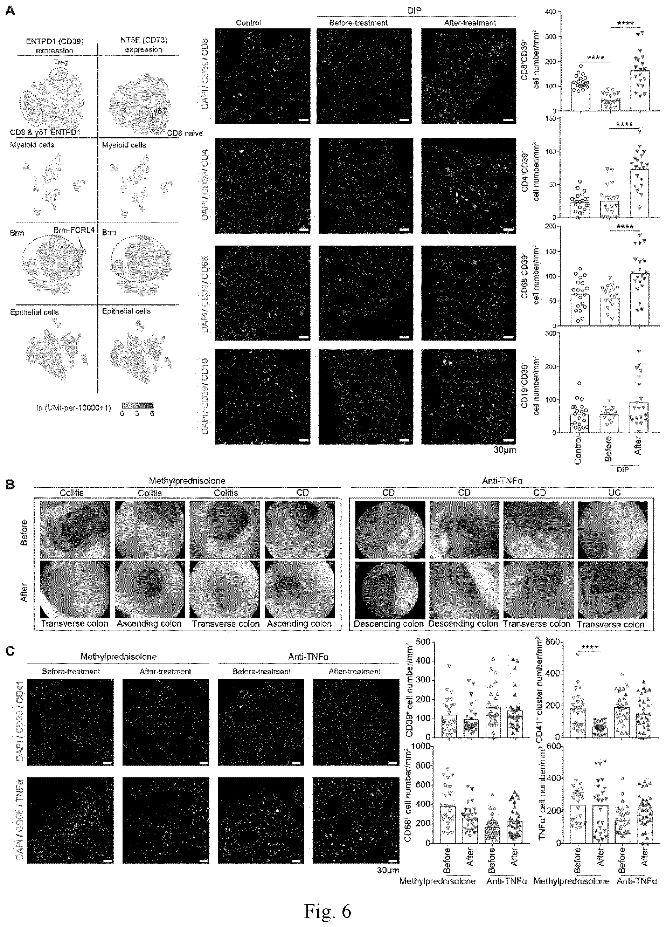

Figure 6. Compared with dipyridamole, methylprednisolone and anti-TNF-α therapy show a different mode of action. (A) The left image, the tSNE image shows the expression of ENTPD1 and NT5E (CD73) in the main cell clusters. Cells are color-coded by normalized UMI counts of selected genes. The subtypes of cells with rich expression of these two genes are shown. In the middle panel, representative immunofluorescence staining shows the changes in the expression of CD39 in T cells, macrophages and B cells in the colonic mucosa after dipyridamole treatment (n=3). In the right panel, quantitative analysis showed that CD39 expression in T cells and macrophages was significantly increased after dipyridamole treatment. For any given subject, colon biopsies are counted in 5 areas. The colors of the dots represent different groups (black represents the control group; green represents colitis). The horizontal line represents the median value. The Mann-Whitney U test was used to calculate the P value. ****, P <0.0001. (B) For children with colitis or IBD, before and after methylprednisolone administration (left), or before and after anti-TNFα administration (right), representative of the same colon segment Looking glass image. (C) Representative immunofluorescence staining shows that for children with colitis or IBD before and after methylprednisolone administration (left, n = 5) or anti-TNF α administration (right, n = 6), changes in the differences of CD39, CD41, CD68 and TNF-α. The quantitative analysis is shown on the right. A colon biopsy was performed on 5 areas of the subject. The color of the dots represents the different groups (black represents the control group, green represents colitis, purple represents ulcerative colitis (UC), and red represents Crohn's disease (CD)). The horizontal line represents the median value. The Mann-Whitney U test was used to calculate the P value. ****, P <0.0001.

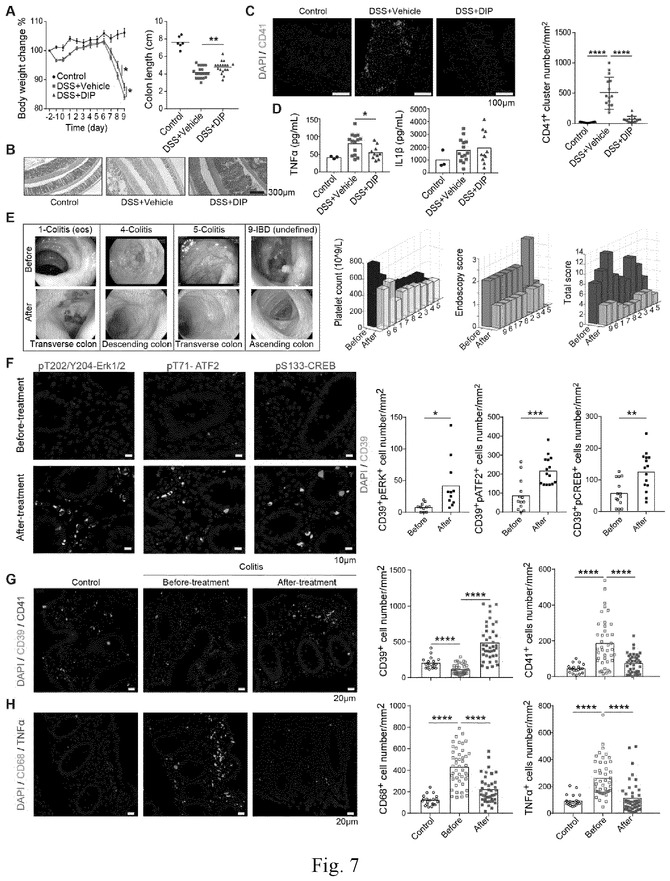

Figure 7: Dipyridamole improves the symptoms of colitis. (A) In the DSS-induced acute colitis model, the body weight and colon length of mice treated with dipyridamole (10 mg/kg) increased significantly (control group, n = 6; DSS + vehicle, n = 23; DSS + dipyridamole, n = 23) P value is calculated by unpaired t-test. *, P <0.05; **, P <0.01. (B) Representative hematoxylin-eosin (HE) stained sections of the colon from the control group, DSS and DSS + dipyridamole (DIP) groups, showing the epithelial integrity and crypt structure of the dipyridamole treatment group Improved (n = 3 per group). (C) Immunofluorescence staining showed that in DSS-induced colitis mice treated with dipyridamole (10 mg/kg) (n = 3 per group), the CD41+ clusters in the colonic mucosa were reduced. The quantitative results are shown in the figure on the right. Colon biopsies were counted in 5 areas. Each point represents an area. The horizontal line represents the median value. The Mann-Whitney U test was used to calculate the P value. ****, P <0.0001. (D) Compared with the DSS group, the expression of TNF-adhesion in the colon mucosa of mice treated with dipyridamole was significantly reduced. The Mann-Whitney U test was used to calculate the P value. *, P <0.05. (E) Left image: Endoscope of the same colon segment before and after dipyridamole administration. The histogram on the right shows the platelet count (109/L), endoscopy score and total clinical score of 9 children before and after dipyridamole treatment. (F) The left image is immunofluorescence staining, showing the activation of ERK (red), ATF10102 (red) and CREB (red) and CD39 (green) co-expressed in colonic mucosa before and after dipyridamole treatment (n = 3 ). Yellow indicates overlap. The figure on the right shows the number of cells co-expressing activated ERK, ATF2 or CREB in the colonic mucosa before (n = 3) and after (n = 3) treatment with dipyridamole. Colon biopsies were counted in 5 areas. Each point represents an area. The horizontal line represents the median value. The Mann-Whitney U test was used to calculate the P value. *, P <0.05; **, P <0.01; ***, P <0.001. (G) Immunofluorescence staining showed that CD39 expression increased and CD41 expression decreased in children's colon mucosa after dipyridamole treatment (n = 7). The color of the dot represents the group (black represents the control group; green represents colitis; blue represents eosinophilic colitis; red represents IBDU). Colon biopsies were counted in 5 areas. Each point represents an area. The horizontal line represents the median value. The Mann-Whitney U test was used to calculate the P value. ****, P <0.0001. (H) Immunofluorescence staining showed that the abundance of CD68+ macrophages in the colonic mucosa of children decreased and the expression of TNF-α decreased after dipyridamole treatment (n = 7). The color of the dot indicates the group (black represents the control group; green represents colitis; blue represents eosinophilic colitis; red, IBDU). Colon biopsies were counted in 5 areas. Each point represents an area. The horizontal line represents the median value. The Mann-Whitney U test was used to calculate the P value. ****,P<0.0001.

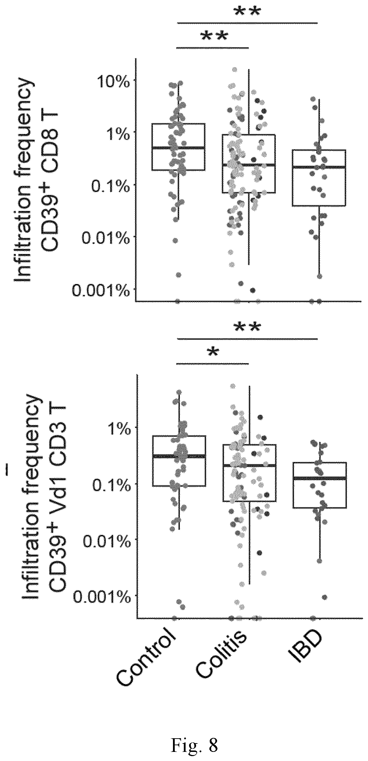

Figure 8: A box plot comparing the frequency of infiltration of CD39 + CD8 T cells (top panel) and CD39 + Vδ1 T cells (bottom panel). Different colors indicate different groups. The Mann-Whitney U test was used to calculate the P value. *, P <0.05; **, P <0.01.

### Embodiments

The gastrointestinal disease described in the present invention is a general term for a variety of inflammatory diseases of the digestive tract, involving the gastrointestinal tract such as stomach, duodenum, small intestine, colon, etc., including: non-infectious and infectious gastrointestinal diseases , Such as inflammatory, allergic, and functional gastrointestinal diseases and gastrointestinal tumors. Stomach diseases such as acute and chronic gastritis, gastric ulcer, gastric polyps, erosive gastritis, peptic ulcer, gastric tumor, duodenal tumor, gastric stromal tumor, duodenal stromal tumor, etc. Small bowel diseases such as small bowel tumors, mesenteric lymphadenitis, interstitial lesions, etc. Colon diseases such as colitis, ulcerative colitis, Crohn's disease, intestinal tuberculosis, colon polyps, colon cancer and other diseases.

Specifically include: inflammatory gastrointestinal diseases, for example, inflammatory bowel disease, colitis, undifferentiated colitis, Crohn's disease (CD), ulcerative colitis (UC), undefined inflammatory bowel disease (Undefined Inflammatory bowel disease), acute colitis, chronic colitis, proctitis, etc.; gastroenteritis, for example, superficial gastritis, ulcerative gastritis, chronic gastritis, chronic gastroenteritis, esophageal stenosis, esophageal atresia, intestinal stenosis, Intestinal atresia, etc.; allergic gastrointestinal diseases, such as gastrointestinal diseases caused by food allergy, such as eosinophilic esophagitis, eosinophilic gastroenteritis, eosinophilic colitis, non-eosinophilic granulocytes Colitis, food protein-induced enterocolitis syndrome, food protein-induced enteropathy, food protein-induced proctocolitis, non-specific chronic colitis, necrotizing enterocolitis, and Hirschsprung-related preoperative and postoperative inflammation. Preferably, colitis, proctitis, undifferentiated colitis, Crohn's disease, ulcerative colitis, acute colitis, chronic colitis; functional gastrointestinal diseases. The above gastrointestinal diseases include infectious and non-infectious ones. In the present invention, preferably non-infectious gastrointestinal diseases, including colitis, inflammatory bowel disease, such as Crohn's disease (CD), ulcerative colitis (UC), undefined inflammatory bowel disease, food allergies Gastrointestinal diseases, such as eosinophilic esophagitis, eosinophilic gastroenteritis, eosinophilic colitis, non-eosinophilic colitis, food protein-induced enterocolitis syndrome, food protein-induced Intestinal disease, food protein-induced proctocolitis, non-specific chronic colitis, necrotizing enterocolitis, Hirschsprung-related preoperative and postoperative inflammation, more preferably inflammatory bowel disease, such as colitis (such as undifferentiated colitis), Proctitis, Crohn's disease, ulcerative colitis, chronic colitis, acute colitis, eosinophilic colitis, non-eosinophilic colitis. In the present invention, colitis is intended to cover various forms of colitis; IBD is intended to cover Crohn's disease, ulcerative colitis, undifferentiated/undefined IBD, and the like.

In the present invention, patients or subjects with gastrointestinal diseases are not restricted by age and gender, and can be children, adults, and elderly people. It is preferably children, such as newborns to 12 years old, 1-6 years old, and the like. The target of the drug for treating gastrointestinal diseases of the present invention can also be other mammals, such as monkeys, cows, horses, pigs, mice, rats, hamsters, rabbits, cats, dogs, sheep and goats.

The treatment of the present invention also includes prevention. For example, due to some disease-related factors, patients who are expected to have a high risk of developing disease but have not yet developed a disease, or patients who have developed a disease but have no symptoms, administer the drug of the present invention, or For patients who are afraid of disease recurrence after treatment of the disease, the drug of the present invention is administered.

The inventors analyzed the T cell subtypes of the colonic mucosa and found that the reduction of T cell CD39 expression is an important mechanism for the pathogenesis of gastrointestinal diseases such as childhood gastrointestinal diseases, such as non-infectious gastrointestinal diseases. In the present invention, single-cell transcriptome sequencing of colonic mucosa was performed using biopsies of children with inflammatory bowel disease, describing a unique subpopulation of immune cells that constitute the mucosal microenvironment. Bioinformatics analysis found that CD39+ CD8T, CD39+γδT, CD39+ NKT (collectively referred to as CD39+IET, or CD39+CD3T) cell subsets decreased, leading to platelet aggregation and serotonin accumulation, and promote gastrointestinal diseases, such as non-infectious gastrointestinal diseases The occurrence of tract diseases. The inventors discovered the cascade pathogenesis of inflammatory bowel disease that reduces the level of cAMP, which leads to the down-regulation of CD39 expression in CD8 + T cells, and induces platelet aggregation and serotonin accumulation. In addition, the present inventors surprisingly found that phosphodiesterase inhibitor drugs can be used to treat gastrointestinal diseases, such as inflammatory bowel disease in children and other patients. This new treatment direction and drug use.

In addition, the present inventors discovered that, for example, in gastrointestinal diseases such as inflammatory bowel disease, TNF-α-expressing macrophages accumulate under the colonic mucosa in large numbers, and PDE4B is also highly expressed on these macrophages. PDE4B is a phosphodiesterase, which reduces the level of intracellular cAMP, which increases the expression of TNF-α by reducing the concentration of cAMP. Therefore, the pathogenesis caused by the impaired cAMP-response pathway also exists in macrophages.

The present invention uses phosphodiesterase inhibitor dipyridamole for treatment in DSS-induced acute colitis model mice. The experimental results show that dipyridamole can increase the cytoplasmic cAMP concentration and inhibit the expression of TNF-α in monocytes and macrophages both in vitro and in vivo. In addition, dipyridamole effectively inhibited platelet aggregation in the colonic mucosa of mice with enteritis. The experimental results support the use of dipyridamole in the treatment of gastrointestinal diseases such as colitis, IBD, UC or CD.

Specifically, the present invention analyzes the phenotype of intraepithelial T cells (IET). The analysis shows that the abundance of CD39-expressing CD8Trm and γδ T cells is reduced in patients with colitis, UC or CD, and the CD39+IET The decrease in abundance is associated with platelet aggregation and serotonin release in the colonic mucosa. The experiments of the present invention show that the lack of CD39+IET in children with colitis, UC and CD can aggravate colon inflammation through platelet aggregation and the release of serotonin. This analysis shows that dipyridamole can increase the expression of CD39 in T cells and macrophages in a manner related to c-AMP-ATF2-CREB activation, thereby inhibiting platelet aggregation.

Furthermore, the present invention has conducted clinical trials to compare colonic mucosa before and after treatment with dipyridamole. The results show that dipyridamole can simultaneously target multiple cell defects, restore immune homeostasis, and improve clinical symptoms. In addition, as a comparison, the present invention also analyzes the colon mucosa before and after the treatment of patients who have been treated with methylprednisolone or anti-TNF-α. These two treatments are currently clinically used in the treatment of colitis. method. The present invention found that some patients with inflammatory bowel disease treated with methylprednisolone improved the clinical status by significantly reducing platelet aggregation in the colonic mucosa. However, because methylprednisolone treatment did not change the invasion of macrophages, nor did it change the expression of TNF-α, some patients became drug-dependent or the treatment was ineffective. On the other hand, although the use of anti-TNF-α antibody biologics for treatment improved the clinical symptoms, it did not restore the various cellular immune deficiencies as described by dipyridamole. Therefore, these results of the present invention indicate that the existing treatment methods such as methylprednisolone or anti-TNF-α lack the ability to restore multifactor immunodeficiency in patients with inflammatory bowel disease, and the phosphate diphosphate of the present invention The esterase inhibitor dipyridamole can achieve such a therapeutic effect.

Therefore, the present invention provides drugs for treating gastrointestinal diseases (hereinafter sometimes referred to as the drugs for treating gastrointestinal diseases of the present invention) and methods, which are specifically as follows.

The present invention can treat gastrointestinal diseases by increasing cAMP levels and/or cGMP levels. For example, by administering drugs or other appropriate means and methods. The drugs and methods for increasing cAMP levels and/or cGMP levels of the present invention are not particularly limited as long as they can increase cAMP levels and/or cGMP levels. As drugs that increase cAMP levels and/or cGMP levels (also referred to herein as cAMP and/or cGMP promoters) include, but are not limited to, phosphodiesterase inhibitors, adenylate cyclase activators, and/or birds Acid cyclase activator (sometimes simply referred to as adenylate cyclase and/or guanylate cyclase activator), etc.

The meaning of the phosphodiesterase inhibitor in the present invention is as known in the art. It is known in the art that phosphodiesterase (PDE) has the function of hydrolyzing the second messenger in the cell (cAMP, cyclic adenosine monophosphate or cGMP, cyclic guanosine monophosphate), and degrades the intracellular cAMP or cGMP, thereby ending these second messengers. The biochemical effects conducted by the messenger. The phosphodiesterase family includes PDE1, PDE2, PDE3, PDE4, PDE5, PDE6, PDE7, PDE8, PDE9, PDE10, PDE11, etc., each with multiple isoenzyme subtypes, for example, PDE4 includes PDE4A, 4B, 4C And 4D and other subtypes.

The phosphodiesterase inhibitors of the present invention include drugs that inhibit any one or more of the phosphodiesterase family, including selective or non-selective phosphodiesterase inhibitors. Including but not limited to PDE1 inhibitor, PDE2 inhibitor, PDE3 inhibitor, PDE4 inhibitor, PDE5 inhibitor, PDE6 inhibitor, PDE7 inhibitor, PDE8 inhibitor, PDE9 inhibitor, PDF10 inhibitor, PDE11 inhibitor, or A variety of inhibitors in the family and drugs that have inhibitory effects on other members of the phosphodiesterase family. Preferable are inhibitors having PDE3, and/or PDE4, and/or PDE5 inhibitory effects. More preferably, PDE5 inhibitors.

Specifically, the phosphodiesterase inhibitor of the present invention is not particularly limited as long as it has an inhibitory effect on phosphodiesterase, and it is known to include nimodipine, vinpocetine, IC86340, IC224, EHNA, BAY60 -7750, IC933, dipyridamole, cilostazol, cilostamide, milrinone, amrinone, enoximone, cyanoguanidine, theophylline, rolipram, piramilast, Roflumilast, cilomilast, apremilast, sildenafil, vardenafil, tadanafil, mitrepanin, udenafil, BRL-50481, IC242, and discoveries based on computer simulations Quinazoline and thiadiazole small molecule compounds S14 and VP1.15, etc. It is known in the art that sildenafil, vardenafil, tadanafil, mitrofluid, dipyridamole, etc. are PDE inhibitors, especially PDE5 inhibitors.

In addition, the meaning of an adenylate cyclase activator and/or a guanylate cyclase activator is known in the art. Adenylate cyclase, abbreviated as AC, is a membrane integral protein that can convert ATP into cAMP, causing a signal response in the cell. Guanylate cyclase, GC for short, can catalyze GTP to produce cGMP. The adenylate cyclase activator and/or guanylate cyclase activator are directed against the immune pathogenesis of gastrointestinal diseases described in the present invention, and can increase cAMP levels and/or cGMP levels and treat gastrointestinal diseases.

In addition, the antiplatelet drug in the present invention is not particularly limited as long as it has an antiplatelet effect. Including but not limited to, for example, drugs that affect platelet activation and expansion, drugs that inhibit platelet aggregation, and the like. For example, thromboxane A2 (TXA2) inhibitors, adenosine diphosphate (ADP) P2Y12 receptor antagonists, including thiophene pyridines and non-thiophene pyridines, thrombin receptor antagonists, 5-hydroxytryptamine (5-HT) receptors Body antagonist, platelet glycoprotein (glycoprotein, GP) IIb/IIIa receptor inhibitors, phosphodiesterase inhibitors. Specifically, it can be aspirin, ticlopidine (Ticlopidine), clopidogrel (Clopidogrel) and prasugrel (Prasugrel), ticagrelor (Ticagrelor), cangrelor (Cangrelor), ticagrelor (Ticagrelor), Vorapaxar (SCH-530348), Atopaxar (E5555), Sarpogrelate, Citalopram, Asemumab, Tirofiban, Dipyridamole, Cilostazol, etc.

The present invention preferably has the following aspects.

In the present invention, dipyridamole is preferably used for the treatment of inflammatory gastrointestinal diseases such as colitis, inflammatory bowel disease, ulcerative colitis, Crohn's disease, and its pharmaceutical use.

In the present invention, dipyridamole is preferably used for the treatment of allergic gastrointestinal diseases such as eosinophilic colitis and non-eosinophilic colitis, and its pharmaceutical use.

The preferred PDE5 inhibitor of the present invention is used for the treatment of inflammatory gastrointestinal diseases such as colitis, inflammatory bowel disease, ulcerative colitis, Crohn's disease, and its pharmaceutical use.

The preferred PDE5 inhibitor of the present invention is used for the treatment of allergic gastrointestinal diseases such as eosinophilic colitis and non-eosinophilic colitis, and its pharmaceutical use.

The preferred antiplatelet agent of the present invention is used for the treatment of inflammatory gastrointestinal diseases such as colitis, inflammatory bowel disease, ulcerative colitis, Crohn's disease, and its pharmaceutical use.

The preferred antiplatelet drugs of the present invention are used for the treatment of allergic gastrointestinal diseases such as eosinophilic colitis and non-eosinophilic colitis, and their pharmaceutical uses.

In each of the above aspects, the disease is infectious or non-infectious.

Those skilled in the art can understand that the form of the active ingredient in each drug for treating gastrointestinal diseases described in the present invention is not limited, and it can be the active compound itself, the free state, its salt, ester, isomer, optically different form. Structure, stereoisomer, regioisomer, geometric isomer, hydrate, non-hydrate, solvate or non-solvate, amorphous, crystal, pharmaceutically acceptable co-crystal or co-crystal salt, derivative, Various forms such as prodrugs. Prodrugs can be converted into the active ingredients in organisms under physiological conditions due to the reaction of enzymes, gastric acid, etc., that is to say, can be converted into active ingredients through enzyme-induced oxidation, reduction, hydrolysis, etc. A compound of an ingredient; a compound that can be converted into an active ingredient by hydrolysis or the like due to gastric acid, etc. Eutectic or eutectic salt refers to a crystalline substance composed of two or more specific substances. At room temperature, each substance is solid and has different physical properties (for example, structure, melting point, heat of fusion, hygroscopicity, Solubility, stability, etc.). Co-crystals and co-crystal salts can be prepared by co-crystallization methods known per se.

For example, the active ingredient in the phosphodiesterase inhibitor (such as dipyridamole) of the present invention is not limited in the form of the active ingredient, and it can be the active compound itself, free form, salt, ester, isomer, optically different form. Constructs, stereoisomers, regioisomers, geometric isomers, hydrates, non-hydrates, solvates or non-solvates, amorphous, crystals, pharmaceutically acceptable co-crystals or co-crystal salts, derivatives, Various forms such as prodrugs.

In the present invention, when the active ingredient is a salt, examples of such salts include metal salts, ammonium salts, salts with organic bases, salts with inorganic acids, salts with organic acids, and basic or acidic amino acids. The salt formed, and so on. Preferable examples of metal salts include: alkali metal salts, for example, sodium salt, potassium salt, etc.; alkaline earth metal salts, for example, calcium salt, magnesium salt, barium salt, etc.; and aluminum salt. Preferable examples of salts with organic bases include salts with the following organic bases: trimethylamine, triethylamine, pyridine, picoline, 2,6-lutidine, ethanolamine, diethanolamine, triethanolamine, cyclohexane Amine, dicyclohexylamine, N,N'-dibenzylethylenediamine, etc. Preferable examples of salts formed with inorganic acids include: salts formed with hydrochloric acid, hydrobromic acid, nitric acid, sulfuric acid, phosphoric acid and the like. Preferable examples of salts with organic acids include salts with the following organic acids: formic acid, acetic acid, trifluoroacetic acid, phthalic acid, fumaric acid, oxalic acid, tartaric acid, maleic acid, citric acid, succinic acid, apple Acid, methanesulfonic acid, benzenesulfonic acid, p-toluenesulfonic acid, etc. Preferable examples of salts with basic amino acids include salts with the following basic amino acids: arginine, lysine, ornithine, and the like. Preferable examples of salts with acidic amino acids include salts with the following acidic amino acids: aspartic acid, glutamic acid, and the like.

Among these, pharmaceutically acceptable salts are preferred. For example, when the active ingredient contains an acidic functional group, examples thereof include inorganic salts, for example, alkali metal salts (e.g., sodium salt, potassium salt, etc.), alkaline earth metal salts (e.g., calcium salt, magnesium salt, etc.), etc. Etc., ammonium salts, etc., when the compound contains a basic functional group, examples thereof include salts formed with inorganic acids, such as hydrochloric acid, hydrobromic acid, nitric acid, sulfuric acid, phosphoric acid, etc., and salts formed with organic acids, such as , Acetic acid, phthalic acid, fumaric acid, oxalic acid, tartaric acid, maleic acid, citric acid, succinic acid, methanesulfonic acid, benzenesulfonic acid, p-toluenesulfonic acid, etc.

For example, the active ingredients in the phosphodiesterase inhibitors and antiplatelet drugs of the present invention can be in free form, salt form, ester form, and other modified forms such as various derivatives and prodrugs, for example, dipyridamole In other words, it can be in free form, ester, salt, derivative, prodrug, and other modified forms.

The active ingredients of the drugs for treating gastrointestinal diseases described in the present invention, such as drugs used to increase cAMP levels and/or cGMP levels and active ingredients of antiplatelet drugs, can be used as the active compound itself or the active compound and the pharmacological agent. It is administered in the form of a mixture of accepted carriers.

As a pharmaceutically acceptable carrier, various organic or inorganic carrier materials commonly used as preparation materials can be used, and are not particularly limited, and can be excipients, lubricants, binders, and disintegrants in solid preparations; in liquid preparations It can be formulated in the form of solvents, solubilizers, suspending agents, isotonic agents, buffers, and pain relievers. In addition, formulation additives such as preservatives, antioxidants, stabilizers, colorants, and sweeteners can also be used as needed.

The preparation form of the drug for treating gastrointestinal diseases of the present invention is not particularly limited, and it can be used as a drug for parenteral administration or oral administration, for example, in the form of liposomes or exosomes encapsulated. The medicine of the present invention may be any of solid preparations such as powders, granules, tablets or capsules, or liquid preparations such as syrups or emulsions. Drugs for the treatment of gastrointestinal diseases can be safely administered in the following forms (e.g., intravenous, intramuscular, subcutaneous, intraorgan, intranasal, intradermal, drops, intracerebral, intrarectal, vaginal, intraperitoneal, tumor Internal, proximal tumor, lesion, etc.): tablets (including sugar-coated tablets, film-coated tablets, sublingual tablets, orally disintegrating tablets, buccal tablets, etc.), pills, powders, granules Agents, capsules (including soft capsules, microcapsules), lozenges, syrups, liquids, emulsions, suspensions, controlled release formulations (for example, immediate release formulations, sustained release formulations, sustained release microcapsules), aerosols Agents, membranes (for example, oral disintegrating membranes, oral mucosal adhesive membranes), injections (for example, subcutaneous injection, intravenous injection, intramuscular injection, intraperitoneal injection), intravenous infusion, transdermal absorption preparations, Creams, ointments, lotions, adhesive preparations, suppositories (for example, rectal suppositories, vaginal suppositories), granules, nasal preparations, lung preparations (for example, inhalants), eye drops, and the like.

The content of the pharmaceutical active ingredient of the present invention in the pharmaceutical composition varies based on the dosage form, dosage, etc. of the compound of the present invention. For example, the content is in the range of about 0.1 to 100 wt%.

The dosage of the drug for treating gastrointestinal diseases in the present invention, such as non-infectious gastrointestinal diseases, is not particularly limited, as long as it is a therapeutically effective amount. In the present specification, the term "therapeutically effective amount" refers to an amount that brings a therapeutic effect to a subject. For example, in a subject to which the amount is administered, the symptoms or symptoms of the disease are compared with those to which the amount is not administered The condition is alleviated, alleviated, or eliminated, or the development of the symptoms or condition of the disease is delayed or suppressed. The therapeutically effective amount can be appropriately determined by the doctor according to the age, weight, sex, and severity of symptoms of the subject. For example, for children, 0.1-100 mg/kg/day, 1-50 mg/kg/day, 3-20 mg/kg/day, one to several times a day.

The medicine for treating gastrointestinal diseases of the present invention can be used in combination with other medicines. The other drugs are for example: anti-atherosclerotic drugs, antithrombotic drugs, anti-heart failure drugs, antiarrhythmic drugs, antihypertensive drugs, drugs for the treatment of diabetes, drugs for the treatment of diabetic complications, drugs for improving HDL, anti- Hyperlipidemia drugs, anti-obesity drugs, diuretics, anti-inflammatory agents, anti-gout drugs, chemotherapeutics, immunotherapeutics such as anti-TNFα drugs, hormones such as glucocorticoid drugs, osteoporosis drugs, anti-dementia Drugs, erectile dysfunction improving drugs, urinary incontinence drugs, and dysuria drugs. These other drugs can be low-molecular compounds or high-molecular proteins, polypeptides, antibodies, vaccines, and the like.

There is no restriction on the administration time of the drug for treating gastrointestinal diseases of the present invention and the other drugs, and they can be administered to the patient simultaneously or in a staggered manner. The dosage of the other drugs can be appropriately determined based on the dosage used in the clinical condition, and can be appropriately determined according to the administration patient, the administration route, the targeted disease, the symptoms, the combination drug, and the like.

The present invention provides a method for diagnosing gastrointestinal diseases. The method includes, for example, detecting the expression of CD39 on T cells and/or the number of platelets. If the expression of CD39 decreases and/or the number of platelets increases, then the method is judged as positive.

The present invention also provides a method for diagnosing gastrointestinal diseases. The method includes, for example, detecting T cell CD39 expression in the intestinal mucosa of a patient or subject, and/or platelet aggregation, and/or expression of TNF-α and/or Infiltration of PDE4B macrophages, if the expression of CD39 decreases, and/or the number of platelets increases, and/or the expression of TNF-α increases, it is judged to be positive.

The present invention provides a kit for diagnosing gastrointestinal diseases, which includes, for example, a reagent for detecting whether T cell CD39 expression is decreased, and/or a reagent for detecting whether the number of platelets is decreased.

The present invention provides a kit for diagnosing gastrointestinal diseases, which includes, for example, a reagent for detecting CD39 expression of T cells, and/or a reagent for detecting platelet aggregation, and/or detecting macrophages expressing TNF-α and/or PDE4B The infiltration reagent.

The present invention provides a method for preventing or treating gastrointestinal diseases, the method comprising administering to a patient an effective amount of cAMP and/or cGMP promoter, and/or antiplatelet drug.

The gastrointestinal disease described in the diagnosis, treatment method or kit of the present invention is preferably selected from inflammatory gastrointestinal disease, allergic gastrointestinal disease, and functional gastrointestinal disease.

The gastrointestinal disease described in the diagnosis, treatment method or kit of the present invention is preferably selected from the group consisting of colitis, inflammatory bowel disease, ulcerative colitis, Crohn's disease, eosinophilic colitis, non-eosinophils colitis.

The gastrointestinal disease described in the diagnosis, treatment method or kit of the present invention is preferably a non-infectious gastrointestinal disease.

The patient with the gastrointestinal disease described in the diagnosis, treatment method or kit of the present invention is preferably a child.

### Examples

The present invention is further explained in detail with reference to the following examples, but the present invention is not limited thereto.

### Example 1. Single cell sequencing and bioinformatics analysis were performed on cells in the colon mucosa of colitis and control children

### I. Research plan:

(1) Obtain the colon mucosal tissues of control and children with non-infectious gastrointestinal diseases, and obtain single-cell suspensions;
(2) After surface staining, use flow cytometry to sort four subgroups of T, B, non-TB lymphocytes, and non-lymphocytes;
(3) Perform single-cell transcriptome analysis for each cell subgroup;
(4) Single-cell sequencing results, analysis of the clustering characteristics of immune cell subpopulations, and establishment of the composition and transcription characteristics of colonic mucosal immune cells;
(5) Through signal pathway enrichment analysis, determine the specific functional characteristics of cell subpopulations. Identify cell type-specific and highly expressed transcription factors, establish a transcription factor regulatory network through correlation analysis with gene expression levels; combine non-infectious gastrointestinal disease susceptibility genes with the transcription characteristics of immune cell subtypes obtained by single-cell sequencing Correspondingly, establish the immune and non-immune cell subtypes affected by susceptibility genes.

### II. Experimental method

### (1) Collection of clinical samples (colon mucosa and peripheral blood).

Collect colon mucosa from children with non-infectious gastrointestinal diseases (non-eosinophilic colitis and eosinophilic colitis: 67 and 23, respectively) and 28 control children's colon mucosa through colonoscopy. The specific grouping method As shown in the table below. The ileocecal, ascending, transverse, descending, sigmoid, and rectal mucosa (approximately 1mm × 1mm×3mm) of each enrolled child were collected; at the same time, anticoagulant peripheral blood (approximately 3mL) of the corresponding child was collected for later use. The above collection process has passed the clinical research ethics review of Guangzhou Women and Children Medical Center. Therefore, children with normal colonoscopy and pathological manifestations who need to undergo colonic polyp resection are used as control children.

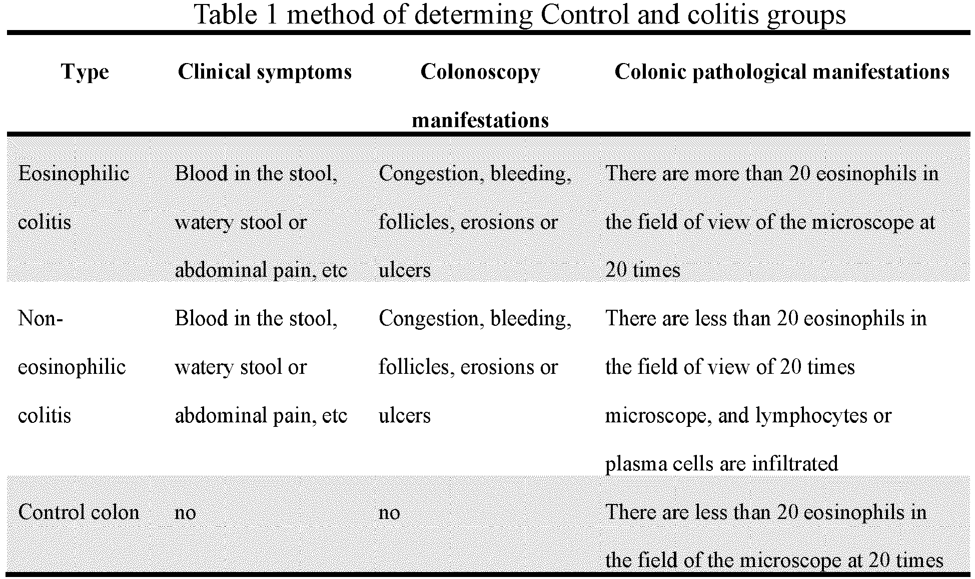

### (2) Extraction of single cells of colonic mucosa.

Cut the colon mucosa with small scissors, and then transfer the tissue into 6ml digestion solution (RPMI1640 contains 10% fetal bovine serum albumin + 10mM HEPES + 100U/ml penicillin + 100µg/ml streptomycin + 1mg/ml collagenase In a centrifuge tube containing 1A+ 10U/ml deoxyribonuclease), shake it horizontally (180RPM, 30 minutes, 37°C), filter with a 70µm filter, centrifuge (900g, 5 minutes), and collect the precipitate for later use.

### (3) Phenotype analysis of T cells.

After treatment (2), the cells are immersed in 60µL flow antibody solution and incubated (20 minutes on ice), then washed once with FACS Buffer (PBS + 2% fetal bovine serum albumin + ImM EDTA), and finally the cells Put it in FACS Buffer (containing 1% PI), and test on flow cytometer.

### (4) Single cell sorting and 5', 3'transcription to build a library.

To collect colonic mucosa and extract single cells of colonic mucosa according to the method (2) of this section, and incubate with antibodies (CD19-APC, CD45-APC-cy7, CD3-FITC, CD4-BV711, CD8α-BV785, CD8β-BV510, γδT) -PE, CD25-PEcy7, CD39-BV421, CD45RA-BV605, PD1-BV650) are sorted by flow cytometry into EP tubes pre-incubated with PBS (containing 5% FBS) for specific sorting and library sequencing The strategy is shown in the table below.

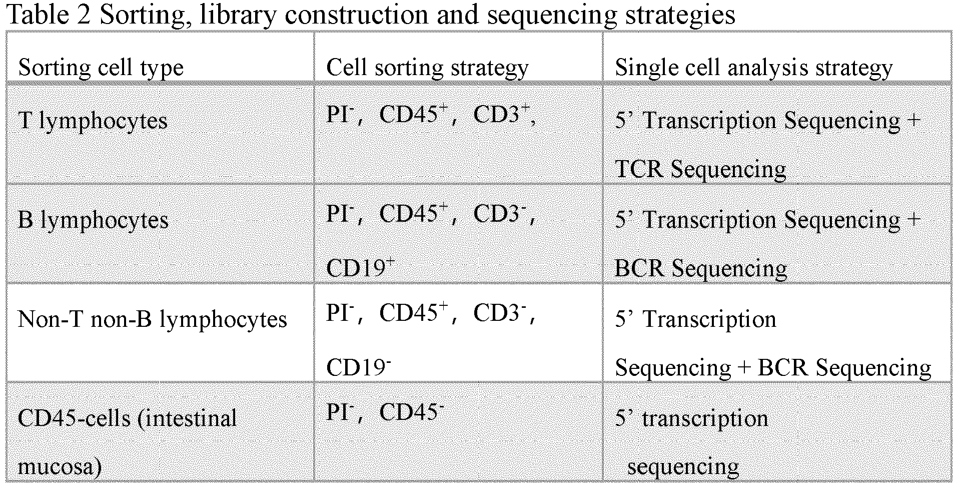

The obtained cells were counted with trypan blue, and then a single cell library was constructed using the 10× Genomic Chroimium Single Cell 5'library building kit. Specifically, according to the kit instructions, the single cell suspension is mixed with the RT-PCR master mix, and then the nano-scale crosslinked microspheres and separation oil are simultaneously loaded onto the special single-cell processing chip. Next, reverse transcribing the RNA in each single cell with specific tags, and purify, amplify, repair the ends and add Illumina specific adapters to the generated cDNA. So far, the single-cell transcriptome is successfully constructed. library. At the same time, on this basis, the Chromium Single Cell V(D)J library building kit was used to amplify the library of TCR and BCR. Finally, the above library was sequenced using Illumina X Ten.

### (5) Intraepithelial T cell phenotype analysis and factor detection.

Place the fresh colon mucosa in normal saline overnight, and use the detection kit (Abcam) for detecting ATP, ADP or Adenosine and the serotonin detection kit (Abcam) to detect the concentration of the corresponding factor in the supernatant. The colonic mucosa was cut into pieces with scissors, and in the digestion solution (PBS containing 5% fetal bovine serum albumin, 10 mM HEPES, ImM dithiothreitol, 100 U/ml penicillin, 100 µg/ml streptomycin and 10 mM EDTA) Shake 180RPM, 37°C, incubate for 40 minutes, and then pass through a 70µm filter to obtain a single cell suspension. By antibody staining (CD45-APC-cy7, CD3-FITC, CD8a-BV785, CD4-BV711, Vd1-PE, Vd2-APC, CD25-PE-cy7, CD127-PE*Dazzle594, D39-BV421, CD69-BV510, CD45RA-BV605 and PD1-BV650) and flow cytometry analysis of phenotype.

### (6) In vitro platelet release serotonin detection experiment

4mL of venous blood is drawn from children with colitis or healthy children, and sodium citrate is used for anticoagulation. After centrifuging the whole blood at 250g and 25°C for 15 minutes, the platelet-rich layer is obtained, which is placed in HBSS (Hank's Balanced Salt Solution) containing 100 nM prostaglandin E1 for further centrifugation at 1500g and 5 minutes, and the platelet pellet is divided into HBSS , The concentration is 109~1010/mL, and incubate overnight at 37°C and 5% CO2. After centrifugation, use the serotonin detection kit to detect the supernatant.

### (7) Immunofluorescence analysis.

Use formalin-fixed and paraffin-embedded paraffin blocks to prepare 3um tissue sections. After deparaffinization, blocking antibodies (PBS containing 5% normal donkey or goat serum and 0.3% Triton X-100) are incubated for 1 hour at room temperature, and washed with PBS Then incubate the primary antibody (CD39, CD41, pT71-ATF2 or pS133-CREB) overnight in a 4C dark humidified box. Then, after washing with PBS, incubate the secondary antibodies (goat anti-mouse IgG1 combined with AF488 and goat anti-rabbit IgG combined with Cy3) for 1 hour at room temperature, and then incubate the anti-quencher containing DAPI. Then, use the Lycra automatic inverted fluorescence microscope to observe and take pictures (20×0.75), and use the Lycra X image analysis software to process the brightness, luminosity, contrast and color balance of the image to restore the image as much as possible. For each observation condition, 4-5 areas in each mucosal section were randomly selected for observation, and the CD41 aggregation (fluorescence diameter greater than 7µm) was measured and counted using the card X image analysis software.

### III. Test results

As shown in Figure 1, through the single-cell sequencing technology, single-cell sequencing and biological information analysis were performed on the cells in the colon mucosa of colitis and control children (a), and a total of 17 T and NK subgroups were found (b) . Among them, the IET expressing ENTPD1 (encoding protein CD39) was significantly reduced in patients with colitis (c) (in figure c, in each group of bars, the left side represents the control group, and the right side represents the colitis group). ENTPD1-CD8 T cells are an immunoprotective cell subtype with specific differentiation characteristics (d), and their differentiation is regulated by the second messenger of cAMP/cGMP, enriching the cAMP response pathway and ERK pathway (e). The cAMP/cGMP second messenger pathway can be induced by phosphodiesterase (PDE) inhibitors or AMP/GMP cyclase activators.

In addition, as shown in Figure 2, the proportion of CD8 and γδT-ENTPD1 cells in children with colitis decreased. Among them, Figures (a) ∼ (c) show the gating strategy and surface marker protein expression in blood and colon intraepithelial T cells (IETs). Panel (d) represents the ratio of designated IETs for control subjects and children with colitis or eosinophilic colitis. In children with colitis, CD39+CD3+T cells, especially CD39+memory CD8T cells (CD39+mCD8T) and CD39+γδT cells (Vδ1+) are significantly reduced in proportion. It is suggested that ENTPD 1+CD8 and γδT defects are related to the pathogenesis of colitis in children.

### Example 2. Clinical experiment and evaluation of dipyridamole in the treatment of colitis in children

The following studies were performed on the colon mucosa of the patients described in Example 1, namely, children with non-eosinophilic colitis and eosinophilic colitis (67 and 23, respectively) and 28 control children. Refer to Table 3 for the patient's condition.

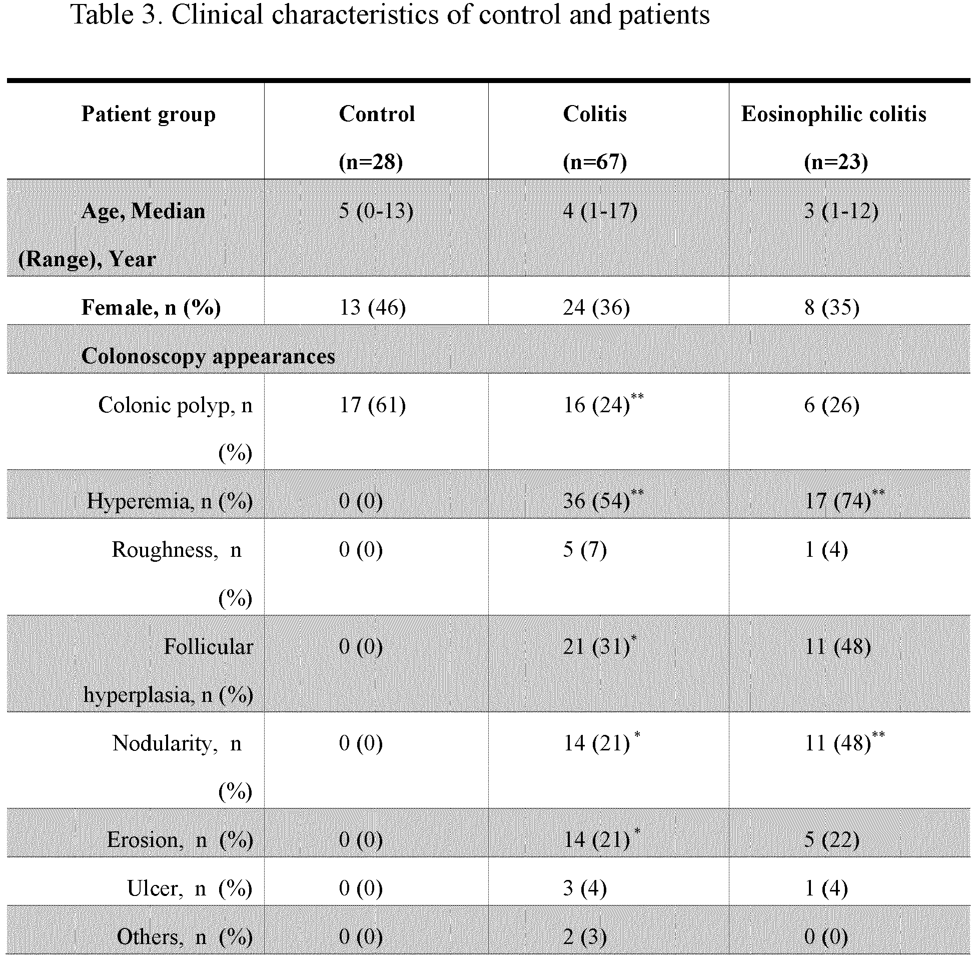

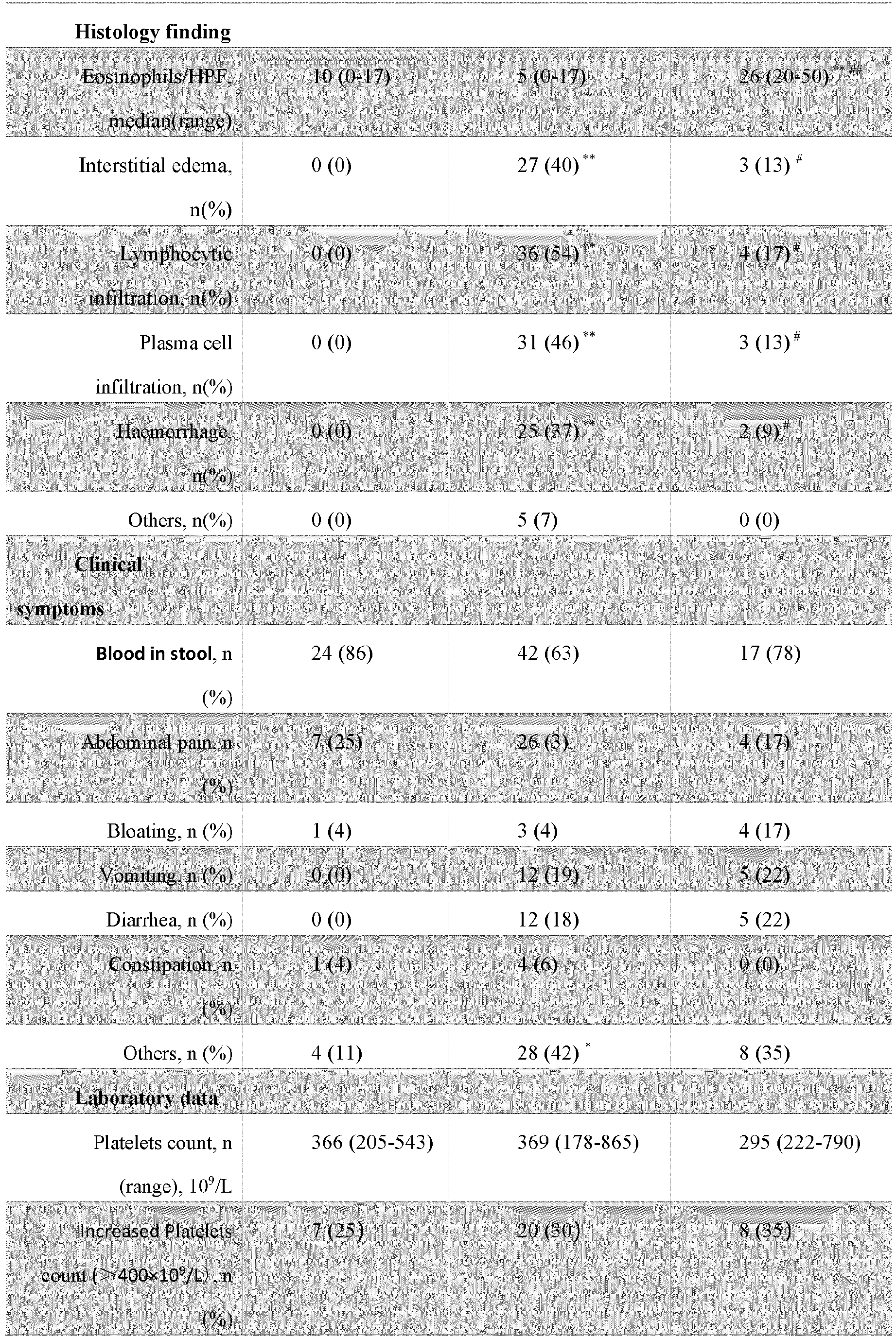

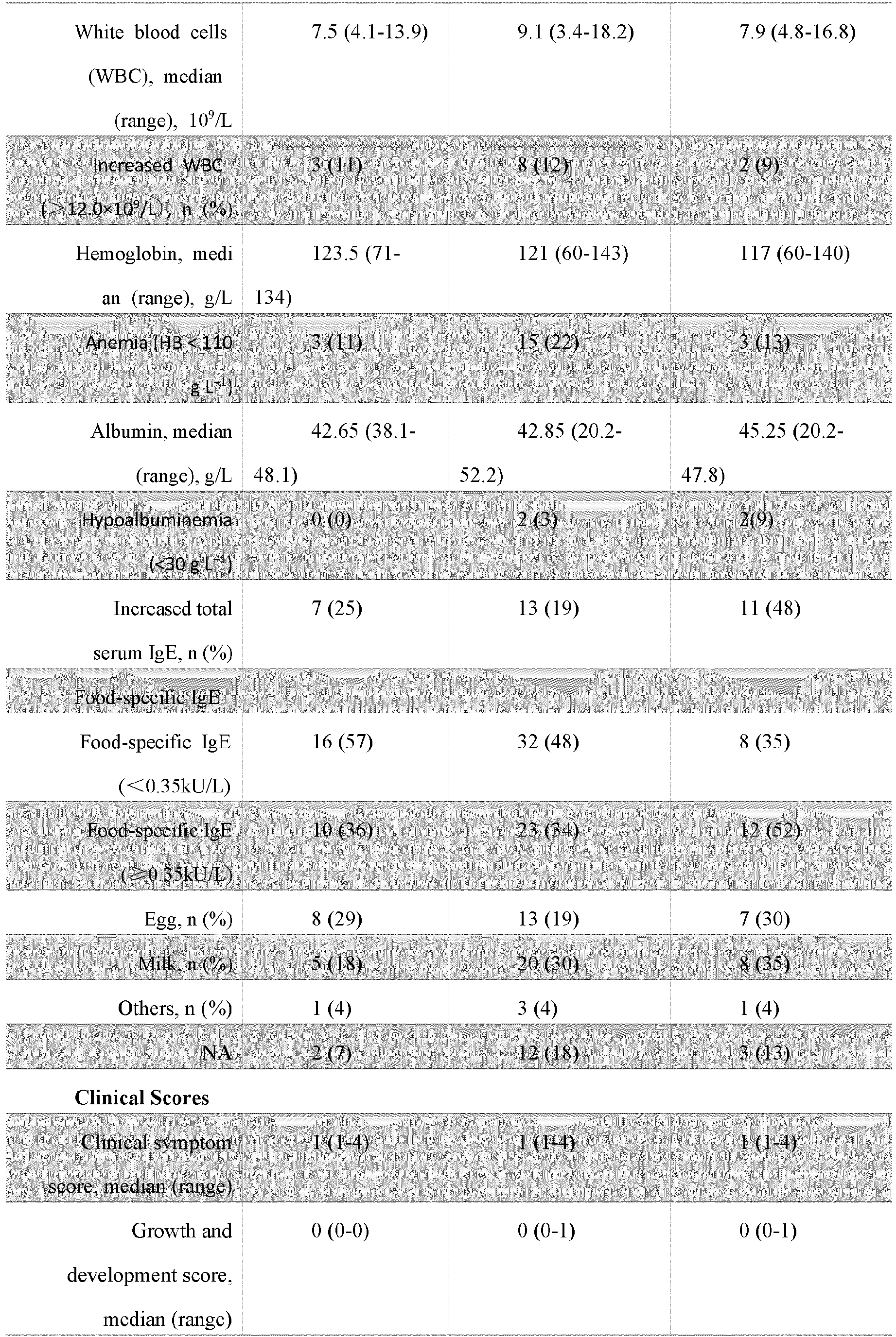

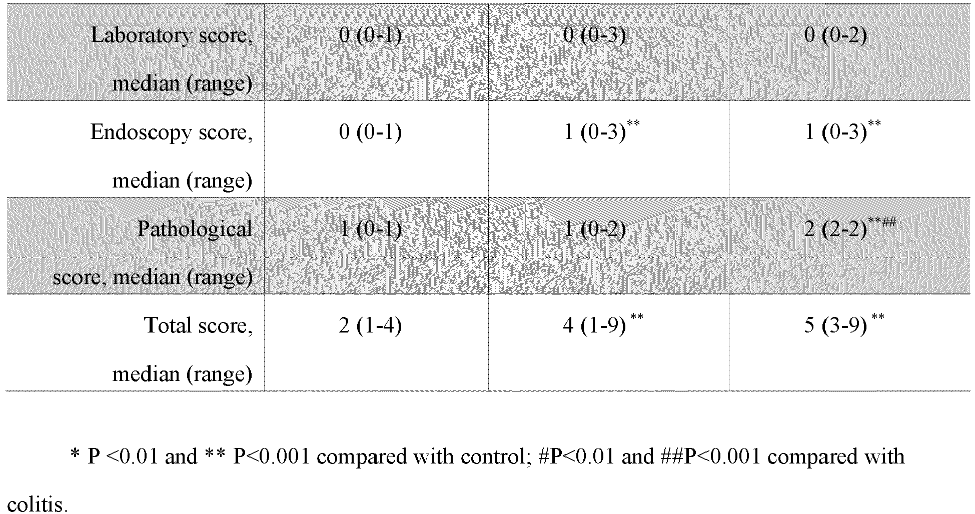

To investigate whether the decrease of CD39 expression in colonic mucosa can aggravate inflammation by promoting the accumulation of ATP and ADP. First, we correlated the expression of CD39 on T cells with ATP and ADP excreted in colon biopsy tissue stored overnight. In these cases, ATP cannot be detected. However, the decrease in CD39 expression corresponds to the increase in the concentration of ADP (Figure 3a)

ADP is an agonist of platelets, and its accumulation and secretion of serotonin correspond to the severity of drug-induced allergic reactions in mice (Beutier et al., 2018). In the release of living tissue from children with colitis, the concentration of ADP and serotonin were positively correlated (Figure 3a). Platelets isolated from the peripheral blood of children with colitis secreted more serotonin than control subjects (Figure 3b). Immunofluorescence staining of colon biopsy showed a negative correlation between CD39 expression and platelet aggregation (measured by CD41+ clusters) in control and colitis subjects, and the number of CD41 clusters in children with colitis was significantly increased (Figure 3c) .

In the following, it is investigated whether the cAMP activation pathway regulates CD39 expression in T cells. It was tested with dipyridamole (a PDE inhibitor and a commonly used antiplatelet drug). Dipyridamole is administered at a dose of 3-5 mg/kg/day for 8-12 weeks. The clinical trial was approved by the Ethical Review Committee of Guangzhou Women and Children's Medical Center and registered in the China Clinical Trial Registration Center (ChiCTR18000l9803). All patients or family members signed an informed consent form.

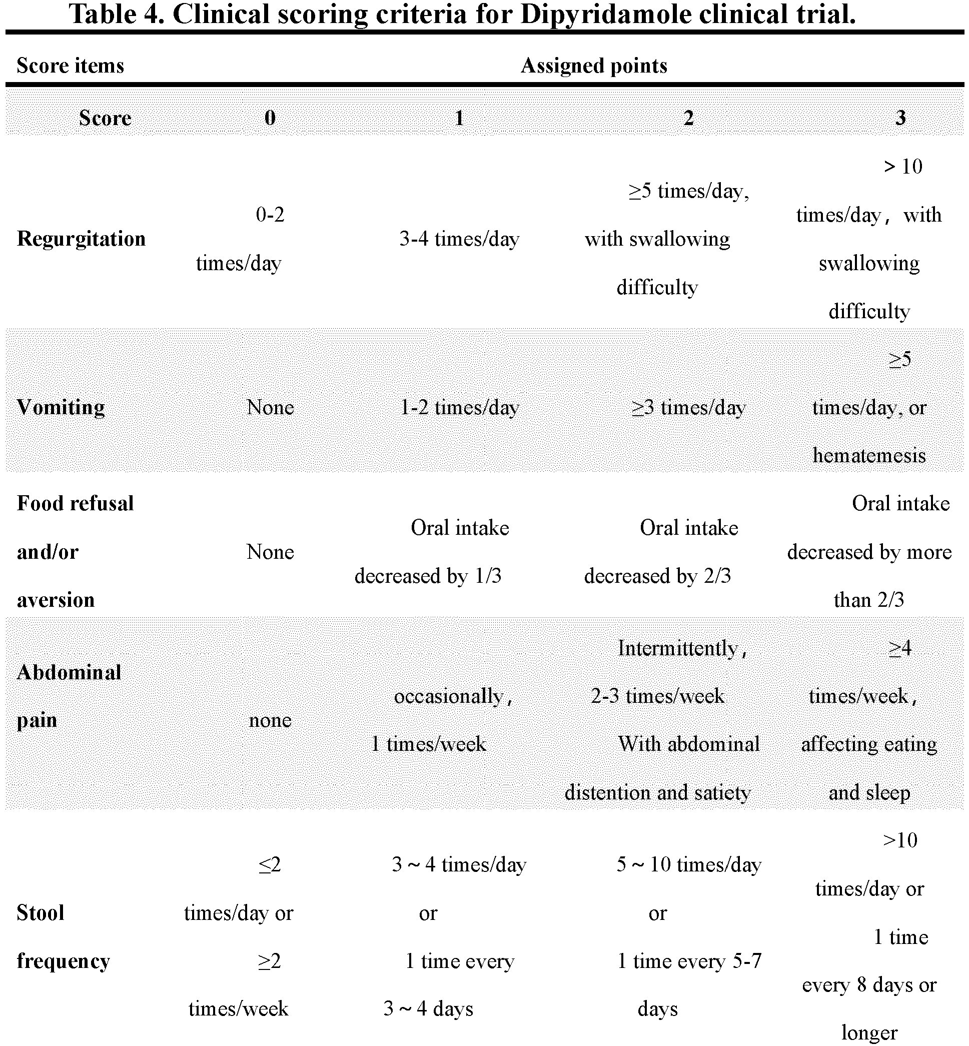

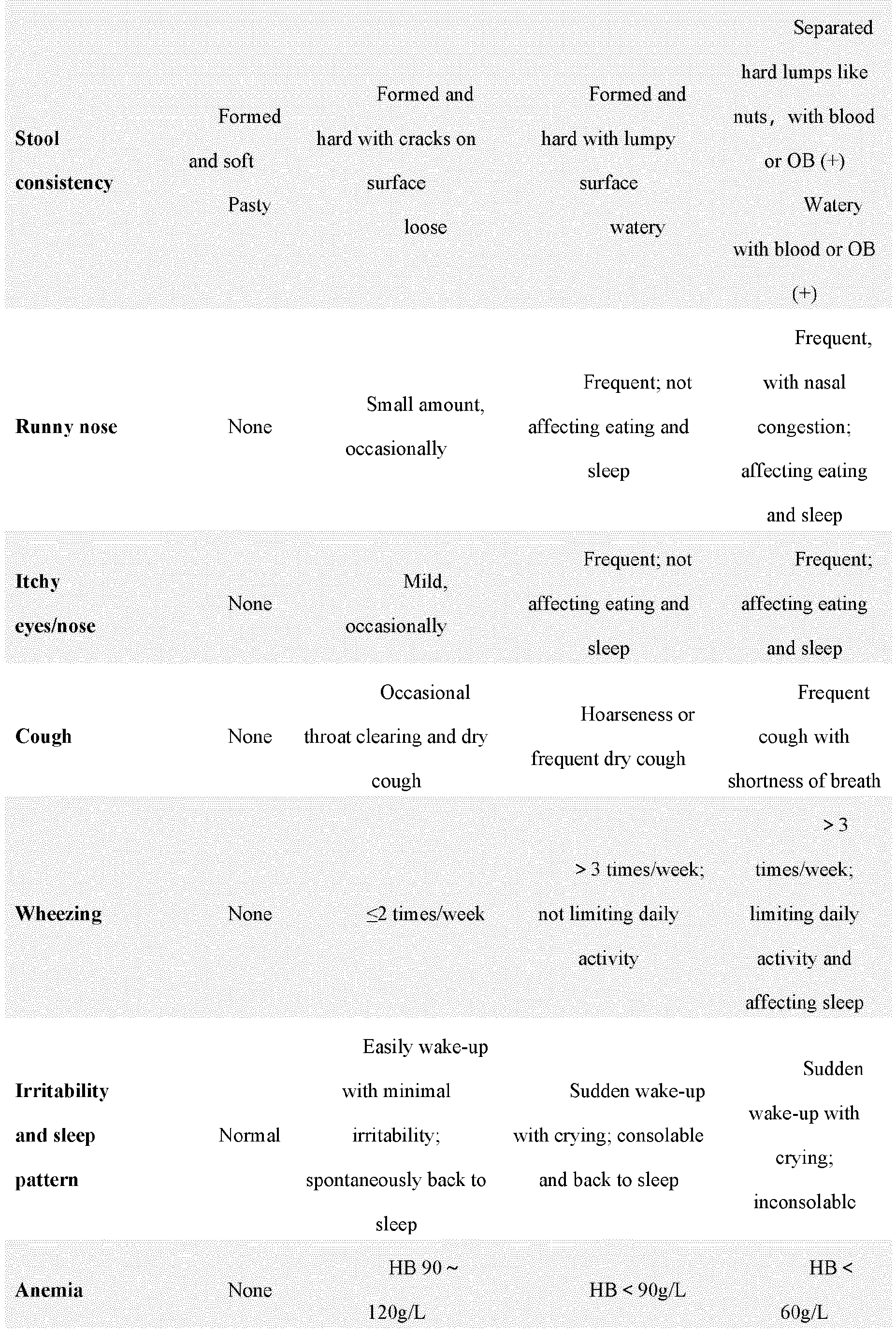

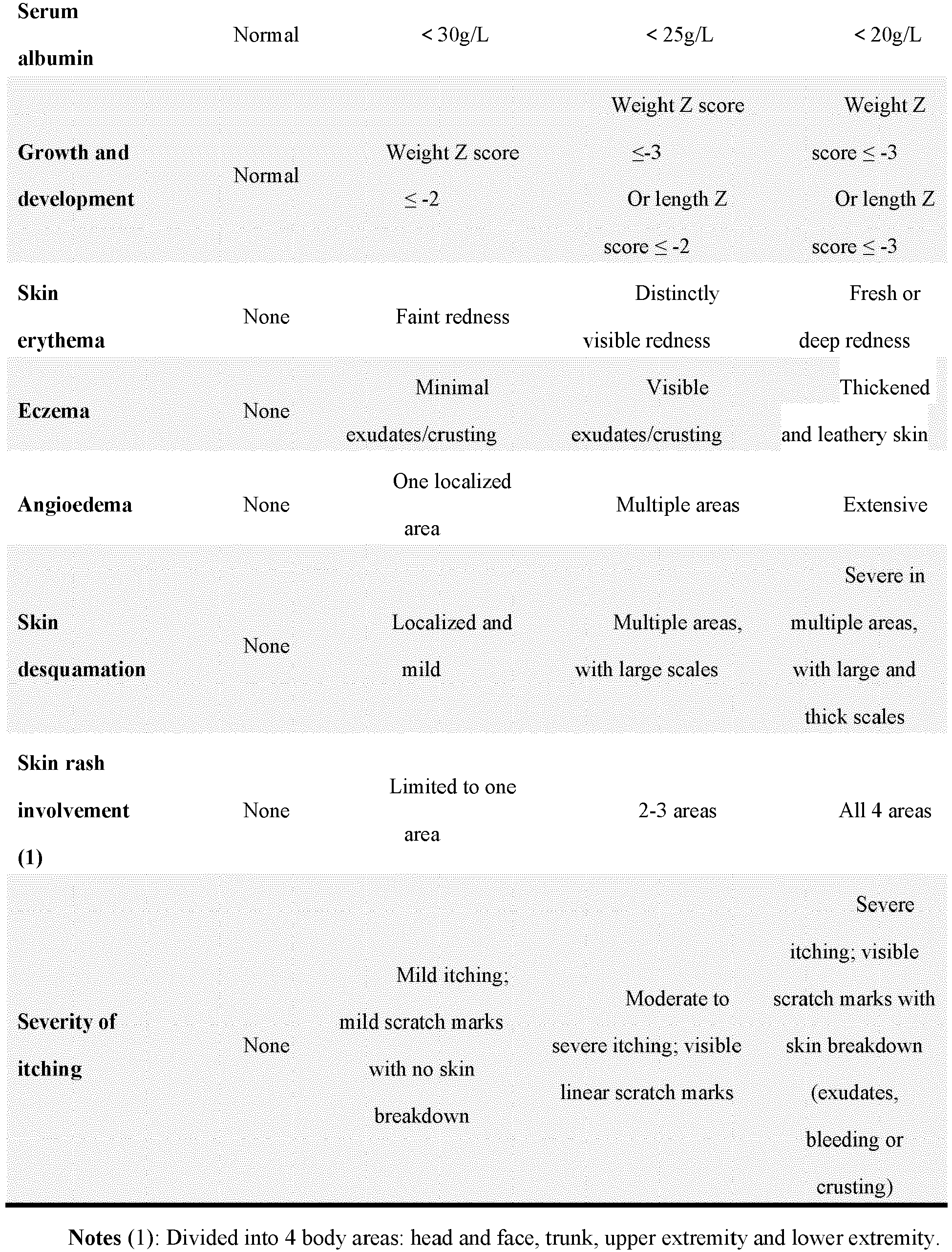

The results of the treatment of Dimitamol are shown in Table 5 below.

**Table 5. Effect of children treated with dipyridamole.**

| **Parameters** | | **Treatment with dipyridamole** |
|---|---|---|
| Number of patients | | 23 |
| Age, median (range), (Year) | | 1.2 (0.3-14) |
| Sex, Male, n (%) | | 17 (74%) |
| WBC, median (range), 10⁹/L | | 8.1 (3.8-16.8) |
| Hemoglobin, median (range), g/L | | 121 (60-138) |
| Food-specific IgE ≥0.35kU/L), n (%) | | 11 (48%) |
| Treatment duration, median (range), weeks | | 4 (2-12) |
| Severity score | | P<0.0001 |
| | Pre-treatment, median (range) | 9 (4-14) |
| | Post-treatment, median (range) | 1 (0-5) |
| Platelet counts | | P=0.0001 |
| | Pre-treatment, median (range) (10⁹/L) (reference | 370 (235-790) |
| range 140-440) | | |
| | Post-treatment, median (range) (10⁹/L) (reference | 296 (217-497) |
| range 140-440) | | |

| | | |
|---|---|---|
| **P value calculated by Wilcoxon matched-pairs signed rank test (2-tailed)** | | |

The results showed that dipyridamole significantly increased CD39 expression during CD8 + T cell activation in a dose-dependent manner. Dipyridamole can restore CD39 expression by activating the cAMP response pathway. From the above results, it is inferred that dipyridamole may target to increase the expression of CD39 and reduce the activity of platelets to improve children's colitis.

After treatment with dipyridamole, there were no reports of adverse reactions, the clinical severity score was significantly reduced, and the colon lining was also significantly improved (Figure 3d); further, immunofluorescence experiments showed that dipyridamole increased in CD8 and CD4 The expression of CD39 in T cells and in macrophages, while the aggregation of platelets in the mucosa was reduced (Figure 3f). At the same time, the co-localized activation transcription factors ATF2 and CREB also increased with the increase of CD39 expression (Figure 3g). This result supports that dipyridamole may promote the up-regulation of CD39 expression through the cAMP signaling pathway.

The inventors also conducted clinical trial studies in subjects with colitis confirmed by endoscopy and subjects with undefined IBD (IBDU) (Table 6). The dose of dipyridamole is 3-5 mg/kg/day for 8 to 12 weeks. No obvious adverse reactions were reported. Before and after the use of dipyridamole, the mucosal healing was evaluated by endoscopy. After dipyridamole treatment, the clinical, endoscopic and histological severity scores were significantly improved (Figure 7E, Table 6). Immunofluorescence staining confirmed that dipyridamole can increase the expression of CD39 in CD8 and CD4 T cells and macrophages (Figure 6A). The enhanced co-expression of activated ATF2 and CREB and CD39 is consistent with the view that increasing cAMP concentration promotes CD39 expression (Figure 7F). The results showed that dipyridamole significantly reduced platelet aggregation (Figure 7G). In addition, macrophage infiltration and TNF-α expression were also significantly reduced after treatment (Figure 7H). In addition, as shown in Figure 4, compared with control subjects, there is a highly inflammatory macrophage and dendritic cell (DC) infiltration in colitis patients.

### Table 6:

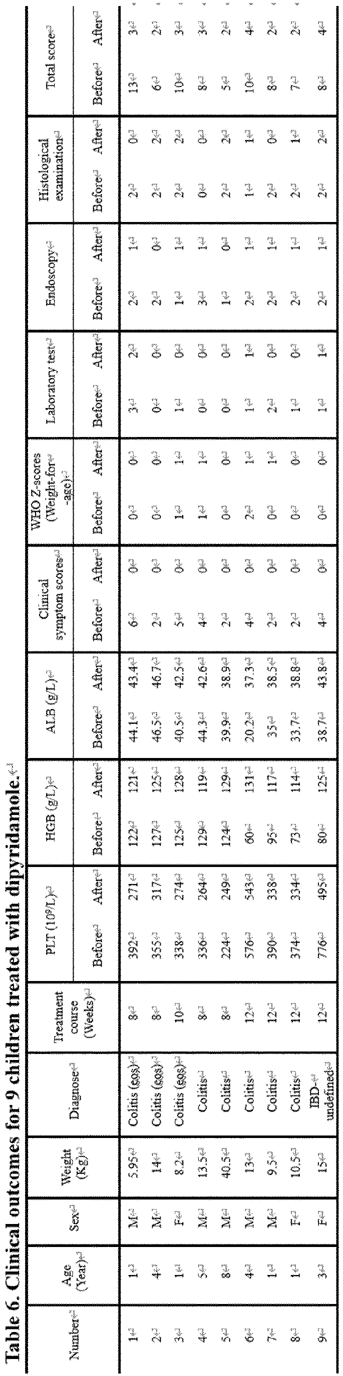

In Table 6, PLT stands for platelet count, HGB stands for hemoglobin, and ALB stands for albumin.

### Example 3

The role of dipyridamole in DSS (dextran sulphate sodium)-induced acute colitis model

### Mouse

Male C57BL/6J mice (20-22 g), six to eight weeks old, were purchased from Guangzhou University of Traditional Chinese Medicine. The mice were given regular food and water before the experiment. Weight-matched mice were used in all studies. Animal research was approved by the Animal Care and Utilization Committee of Guangzhou Medical University (permit number: 2019-471) and was conducted in accordance with institutional guidelines.

### DSS-induced acute colitis

Mice were pretreated with dipyridamole (10 mg/kg body weight, Sigma-Aldrich) or vehicle (2% DMSO, 10% ethanol, 88% corn oil) twice a day for 3 days, and then DSS (36,000 -50,000 MW), MP Biomedicals) added to their drinking water (3%) for 9 days. The control mice were given regular food and water. The mice were monitored daily and if the weight loss exceeded 20%, they were euthanized.

For hematoxylin and eosin (H&E) staining, the colon was fixed in 4% paraformaldehyde (PFA) for 24 hours and embedded in paraffin. Prepare sections (3µm) and deparaffinize before staining.

To detect platelet aggregation in colon slices, the tissue was fixed in 4% PFA for 24 hours, cryoprotected in 30% sucrose for 48 hours, and then embedded in an Optimal Cutting Temperature (O.C.T.) compound. The frozen sections (5µm) were washed, blocked with 10% goat serum, and incubated with anti-mouse CD41-FITC (133903, BioLegend, CA, USA) at 4°C overnight.

After washing with PBS to remove unbound antibodies, the sections were fixed with VECTASHIELD anti-fading fixation medium (H-1200, Vector Laboratories, CA, USA) with DAPI for nuclear staining. The immunofluorescence staining and image processing were performed as described above. Platelet aggregates are defined as CD41+ clusters (diameter> 7µm). Leica X image analysis software (Leica, Hamburg, Germany) and ImageJ software (National Institutes of Health, Maryland, USA) were used to count and analyze the number of CD41 + clusters per square millimeter. For each colon section, platelet aggregation in 5 randomly selected areas was recorded.

In order to analyze the expression of cytokines, the residual mesenteric fat in the colon was removed, weighed and dispersed in a single cell suspension with 1 mL of PBS. After centrifugation at 1000×g for 10 minutes at 4°C, the supernatant was taken to determine the concentration of TNF-α, IL-1β and IL-6 by ELISA.

### CD39 expression of immune cells in DSS colitis model

To determine CD39 expression in colonic immune cells, mice were treated with vehicle, low-dose dipyridamole (5 mg/kg) or high-dose dipyridamole (50 mg/kg) for 8 days, as shown in Figure 5A. From the second day of treatment, DSS was added to drinking water (2.5%) for 7 days. The control mice were given regular food and water.

In order to analyze colon immune cells, the present invention harvests the colon and removes excess fat. After washing with ice-cold PBS, the colon was cut into pieces approximately 0.3-0.5 cm in length. After gentle rotation in the dissociation solution (HBSS without Ca/Mg, with 5 mM EDTA and 2% FBS) at 37°C for 40 minutes, the intraepithelial lymphocytes (IEL) were pelleted, washed and resuspended in staining buffer (PBS 2 %FBS).

The remaining undigested colon tissue samples were gently rotated in the digestion solution (RPMI1640 containing 2% FBS, 2 mg/mL collagenase IV, 0.4 U/mL dispase and 1 µg/mL DNase) at 37°C for 45 minutes. Pellet lamina propria lymphocytes (LPL), wash and resuspend in staining buffer. T cell plates (CD45-APC-Cy7, CD3-BV711, CD39PE-Cy7, CD8-BV785, γδT-BV421 and CD4-APC) or myeloid plates (MHC II-BV550, CD11b- BV510 and CD39-PE-Cy7) were stained on ice for 30 minutes, and then analyzed on a FACSAria SORP flow cytometer (BD Science).

It was found that in mice treated with dipyridamole, the colonic epithelial CD4, CD8 and γδ T cells had significantly increased CD39 expression in a dose-dependent manner (see Figure 5). Compared with mice treated with DSS alone, mice with dipyridamole significantly increased body weight and colon length, improved epithelial integrity and structure, and reduced platelet aggregation and TNF-α expression (see figure 7A-D).

### Example 4

### Methylprednisolone (Medrol) or anti-TNF-α drugs (Infliximab) treat colitis and IBD.

In order to compare with children receiving conventional prescription treatment, the present invention analyzes children receiving methylprednisolone (n = 5, 1-1.5 mg/kg per day for 2-4 weeks, and then continuously reduce the dose until receiving treatment Colon biopsy) or anti-TNF-α antibody (n = 6, 5-10 mg/kg at 0, 2 and 6 weeks, then every 8 weeks for 1 to 2 years) . Biopsy was performed 4-7 months before and after methylprednisolone treatment and 3-6 months before and after anti-TNF-α antibody treatment.

The experimental results are shown in Figure 6C. Methylprednisolone significantly reduces the platelet aggregation in the colonic mucosa, but it does not change the expression of CD39 or TNF-α. The effect of methylprednisolone on platelet aggregation also supports what the present invention points out that targeted platelet aggregation can alleviate the symptoms of colitis and IBD in children.

Regarding anti-TNF-α treatment, colonoscopy showed that the treatment improved the appearance of the colon (Figure 6B), but it had no effect on the recovery of various immune deficiencies in macrophages, T cells or platelets. (Figure 6C).

The above experiments show that dipyridamole can improve colon immune homeostasis by simultaneously targeting multiple immune deficiencies. Therefore, drugs with such activity on the cAMP pathway can have a wide range of applications in children and adults suffering from gastrointestinal diseases such as colitis and IBD. However, drugs such as methylprednisolone or anti-TNF-α drugs alone cannot achieve the effect of dipyridamole shown in the present invention.

Combining the various experiments of the present invention, at least the following immunological results are shown:
For untreated patients, Figure 1C, Figure 2D, and Figure 8 of the present invention reflect the reduction of CD39 IET; Figure 3C, 3F, Figure 7C, and Figure 7G reflect platelet aggregation in patients; Figure 4A, Figure 4C, Figure 7D, Figure 7H reflects the patient's macrophage infiltration, high expression of PDE4B, and high expression of TNF; Figure 1C, IE, and Figure 4A reflect the reduction of cAMP in the patient;
For the treatment with dipyridamole, Figure 5, Figure 6A, Figure 7F, and Figure 7G reflect the increase in CD39 after dipyridamole treatment; Figure 7C and 7G reflect the decrease in platelets after treatment; Figure 7H, 4B reflect the treatment After macrophages decreased, PDE4B was inhibited, and TNF-α was inhibited; Figure 4B reflects the increase in cAMP after treatment;
Regarding the effect of methylprednisolone (Figure 6B-C), Figure 6C reflects that it has no effect on CD39 IET, macrophage invasion, PDE4B, and TNF-α, but it is effective in inhibiting platelet aggregation.

Regarding the therapeutic effect of anti-TNF-α (Figure 6B-C), Figure 6C reflects that it has no effect on CD39 IET, platelet, and macrophage invasion.

In summary, the present invention found that, in terms of immune mechanism, the lack of CD39 + IETs is related to platelet aggregation and immune pathogenesis in the subgroup of colitis patients, and can be used as a biomarker for the diagnosis and treatment of non-infectious gastrointestinal diseases in children Things. Phosphodiesterase inhibitors, such as dipyridamole, can improve the level of cAMP/cGMP, improve CD39 + IETs, and improve a variety of immune deficiencies to achieve immune homeostasis. The above results indicate that dipyridamole targets this pathway to improve children's colitis and provide new ideas for the treatment of this disease. At the same time, the present invention found that through anti-platelet aggregation, for example, administration of dipyridamole and methylprednisolone can treat gastrointestinal diseases.

The present invention includes various changes in its scope, and these changes do not deviate from the scope of the present invention. In addition, all situations that are obviously considered to be modifications of the present invention to those skilled in the art are included in the scope of the claims of the present invention.

### Industrial applicability

The present invention systematically studies the pathogenesis of gastrointestinal diseases. In patients with gastrointestinal diseases, there are defects such as highly inflammatory macrophage infiltration, CD39+IET deficiency and platelet aggregation, and the defective cAMP response pathway serves as a common The mechanism works. The discovery of this mechanism provides new ideas for drugs for gastrointestinal diseases. Further, the present invention targets this mechanism and uses the PDE inhibitor dipyridamole to treat patients with gastrointestinal diseases, and achieves better therapeutic effect results than the existing therapeutic drugs methylprednisolone and anti-TNF-α drugs.

## Claims

1. Use of antiplatelet drugs in the preparation of drugs for the treatment of gastrointestinal diseases.

2. The use of claim 1, wherein the antiplatelet agent is selected from the group consisting of thromboxane A2 inhibitors, adenosine diphosphate P2Y12 receptor antagonists, thrombin receptor antagonists, and serotonin receptor antagonists , Platelet glycoprotein II b/IIIa receptor inhibitors, and phosphodiesterase inhibitors.

3. The use of claim 2, wherein the antiplatelet drug is dipyridamole.

4. The use according to any one of claims 1 to 3, wherein the gastrointestinal disease is colitis or inflammatory bowel disease.

5. The use according to any one of claims 1 to 4, wherein the patient with gastrointestinal disease is a child.

6. Use of cAMP and/or cGMP promoter in the preparation of medicines for the treatment of gastrointestinal diseases.

7. The use according to claim 6, wherein the cAMP and/or cGMP promoter is a phosphodiesterase inhibitor.

8. The use according to any one of claims 6 or 7, wherein the phosphodiesterase inhibitor is dipyridamole.

9. The use according to any one of claims 6-8, wherein the gastrointestinal disease is colitis or inflammatory bowel disease.

10. The use according to any one of claims 1-9, wherein the patient with gastrointestinal disease is a child.
